# EUROPEAN PATENT SPECIFICATION

(11) **EP 4 217 708 B1**
(45) Date of publication and mention of the grant of the patent: **08.04.2026**
(21) Application number: 21802852.0
(22) Date of filing: 24.09.2021
(51) Int. Cl.: G01N 15/1429, G01N 15/14, G01N 15/12, G01N 15/10

(54) **SYSTEMS AND METHODS FOR EVALUATING MIS-C ASSOCIATED WITH COVID-19**
SYSTEME UND VERFAHREN ZUR BEWERTUNG VON MIS-C IM ZUSAMMENHANG MIT COVID-19
SYSTÈMES ET MÉTHODES D'ÉVALUATION DE MIS-C ASSOCIÉ AU COVID-19

(30) Priority: 25.09.2020 US 202063083519 P
(43) Date of publication of application: 02.08.2023
(73) Proprietor: Beckman Coulter, Inc., Brea, CA 92821 (US); The General Hospital Corporation, Boston MA 02114 (US)
(72) Inventor: KEHOE, Sarah, Brea, CA 92821 (US); HASAN, Mohamad Shakil, Brea, CA 92821 (US); XU, Gang, Brea, CA 92821 (US); IRIMIA, Daniel, Boston, MA 02114 (US); YONKER, Lael, Boston, MA 02114 (US)
(74) Representative: Maiwald GmbH
(86) International application number: PCT/US2021/051999
(87) International publication number: WO 2022/067068

(56) References cited:
- CARTER MICHAEL J ET AL: "Peripheral immunophenotypes in children with multisystem inflammatory syndrome associated with SARS-CoV-2 infection", NATURE MEDICINE, vol. 26, no. 11, 18 August 2020 (2020-08-18), pages 1701 - 1707, XP037297421, ISSN: 1078-8956, DOI: 10.1038/S41591-020-1054-6
- GRUBER CONOR N ET AL: "Mapping Systemic Inflammation and Antibody Responses in Multisystem Inflammatory Syndrome in Children (MIS-C)", CELL, ELSEVIER, AMSTERDAM NL, vol. 183, no. 4, 14 September 2020 (2020-09-14), pages 982, XP086341438, ISSN: 0092-8674, [retrieved on 20200914], DOI: 10.1016/J.CELL.2020.09.034
- KOMIYA A ET AL: "Neutrophil CD64 is upregulated in patients with active adult-onset Still's disease", vol. 41, no. 2, 1 January 2012 (2012-01-01), pages 156 - 158, XP009507344, ISSN: 0300-9742, Retrieved from the Internet <URL:http://www.informahealthcare.com/journal/rhe> [retrieved on 20180808], DOI: 10.3109/03009742.2011.644325

## Description

### BACKGROUND

In the early days of the COVID-19 pandemic, it appeared that children were spared from the disease. However, an increasing number of children worldwide have been reported to develop a rare but severe complication of SARS-CoV-2 exposure called Multisystem Inflammatory Syndrome in Children (MIS-C). Most of the MIS-C cases are reported in geographical areas of high COVID-19 incidence, and most cases occur approximately 3-4 weeks after the local peak of the pandemic. Initially reported as hyperinflammatory shock and "Kawasaki-like" illness, MIS-C has been observed to have overlapping features with toxic shock syndrome, atypical Kawasaki disease, macrophage activation syndrome, and cardiogenic and septic shock. Dilated coronary arteries have been reported in 17-25% of the patients with rare development of coronary artery aneurysms and cardiac collapse. Although most MIS-C patients survive with adequate intensive care, deaths have been reported. The long-term consequences of MIS-C are currently unknown.

Identifying MIS-C is challenging. The primary symptom of MIS-C is fever. However, fever is also one of the most common symptoms prompting evaluation by a pediatrician. By the current CDC guidelines, fever in the setting of any SARS-CoV-2 exposures within the past 4 weeks should prompt suspicion for MIS-C. However, potential SARS-CoV-2 exposure is near-universal, given its community spread. To assess for MIS-C, laboratory evaluation is recommended, including complete blood count (CBC), C-reactive protein (CRP), erythrocyte sedimentation rate (ESR), fibrinogen, procalcitonin, d-dimer, ferritin, lactic acid dehydrogenase (LDH), interleukin 6 (IL-6), and albumin. However, many of these labs are non-specific, e.g., elevated neutrophil count on CBC, or elevated CRP. Other laboratory tests take time to be completed, including IL-6, and therefore are less informative at the time of critical clinical decision making. Overall, the assessment of MIS-C could take up to 24 hours and require intensive use of laboratory resources. Accordingly, a rapid, informative detection technology is needed. Carter, M.J., Fish, M., Jennings, A. et al. Nat Med 26, 1701-1707 (2020) describe peripheral immunophenotypes in children with multisystem inflammatory syndrome associated with SARS-CoV-2 infection.

### BRIEF SUMMARY

The invention provides an automated system for evaluating Multisystem Inflammatory Syndrome in Children (MIS-C) associated with SARS-CoV-2 according to claim 1, the system comprising:
a light source (312) configured to irradiate cells of a body fluid sample;
one or more light scatter detector units (350A) configured to measure light scattered from the light source scattered by the cells in the body fluid sample;
a data processing module (304) comprising a processor and a non-transitory computer readable medium, wherein the non-transitory computer readable medium stores instructions that, when executed by the processor, configure the processor to perform a method comprising:
   analyzing a monocyte or granulocyte cell population parameter determined for the body fluid sample based on data from the one or more light scatter detector units; and
   evaluating MIS-C associated with SARS-CoV-2 based at least in part on the monocyte or granulocyte population parameter.

This disclosure relates to a system for evaluating MIS-C associated with COVID-19 in pediatric patients. The system may comprise a flowcell with a flow of a liquid containing a plurality of cells through the flowcell. The system may comprise one or more sensors for detecting light scatter, light transmission, electrical impedance, RF conductivity, or combinations thereof as cells pass through the flowcell. The system may comprise a processor for calculating cell population parameters based on a plurality of measurements of individual cells of the same or related types. The processor may evaluate MIS-C based at least in part on one or more of the cell population parameters.

The invention also provides a method for evaluating MIS-C associated with SARS-CoV-2 according to claim 4, the method comprising:
flowing a body fluid sample through a flowcell;
irradiating a plurality of cells in the body fluid sample in the flowcell;
measuring light scatter from individual cells of the plurality of cells;
analyzing a monocyte or granulocyte cell population parameter determined for the body fluid sample based on the light scatter; and
evaluating MIS-C associated with SARS-CoV-2 based at least in part on the monocyte or granulocyte population parameter.

The method may comprise flowing a body fluid sample through a flowcell, irradiating a plurality of cells in the body fluid sample in the flowcell, measuring light scatter and direct current impedance from individual cells of the plurality of cells, analyzing one or more of the cell population parameters, and evaluating MIS-C associated with SARS-CoV-2 based at least in part on the one or more of the cell population parameters.

One or more cell population parameters can relate to a cell population selected from the group consisting of red blood cells (RBC), white blood cells (WBC), including neutrophils (Ne), early granulocytes (EGC), monocytes, eosinophils (Eo), basophils, and lymphocytes (Ly).

Depending on the technology used, the parameters will vary. In Beckman Coulter's DxH hematology analyzers (including but not limited to the DxH 800 and DxH 900), the WBC differential sample is directed through the flow cell and analyzed with a multi-transducer module composed of a diode laser module, flow cell and two optical sensor assemblies. At least seven parameter measurements are performed that characterize cellular size, shape and morphology. The seven parameters - impedance, radio frequency, and five laser light scatter measurements - are captured simultaneously for each cell passing through the flow cell. These parameters generally measure volume, conductivity, and scatter (VCS).

In addition, each parameter measurement includes four pulse shape attributes. These measurements plus time provide a total of 29 distinct measurements per cellular event. The impedance measurement (volume) is an indicator of three-dimensional cell size. The radio frequency measurement (conductivity) provides information regarding the internal structure of the cell, such as cellular density. In addition to the volume and conductivity measurements, five light scatter measurements - axial light loss (ALL or a derivative thereof, e.g., AL2), low angle light scatter (LALS), lower median angle light scatter (LMALS), upper median angle light scatter (UMALS), and median angle light scatter (MALS) - define the granularity and lobularity characteristics of each cellular event.

The DxH systems use high-speed, high-resolution analog-to-digital conversion with Digital Signal Processing (DSP) circuitry to measure multiple parameters for each cellular event. DSP algorithms analyze the cellular data digitally, providing cellular definition and resolution. Differential accuracy and flagging technology are obtained by combining the additional light scatter measurements with data analysis techniques to further define and separate cell populations. For example, in some cases, MALS may be transformed to a modified rotated MALS (RMALS) parameter through a mathematical transformation to eliminate overlap and create distinct neutrophil, lymphocyte, monocyte and eosinophil populations, enabling optimal analysis and visual confidence in the results. Current internal and visual differential data transformations include: RMALS (rotated MALS), opacity (conductivity minus the size aspect), SOP (stretched opacity), and non-linear AL2 transformations. The DxH systems report "VCS parameters".

Other hematology analyzers, such as those manufactured by Abbott, Sysmex, and Mindray, use the cluster of differentiation (CD) system to differentiate white blood cell populations. CD molecules can act in numerous ways, often acting as receptors or ligands; by which a signal cascade is initiated, altering the behavior of the cell. Some CD proteins do not play a role in cell signaling, but have other functions, such as cell adhesion. The CD system nomenclature commonly used to identify cell markers thus allows cells to be defined based on what molecules are present on their surface. There are more than 350 CD molecules identified for humans. For example, monocytes can be identified with CD45+ and CD14+. Using fluorescently labeled antibodies, these cell markers can used to sort cells. Fluorescence activated cell sorting (FACS) provides a method of sorting a heterogeneous mixture of cells into two or more containers, a single cell at a time, based upon the specific light scattering and fluorescent characteristics of each cell. Fluorescence flow cytometry or FACS may also provide information about cell composition of a labeled cell. For example, information about cell density or complexity may be obtained by measuring light scattered by the cell and information about cell size and internal structure may be obtained by measuring the fluorescence signal intensity of the cell. Systems based on fluorescence flow cytometry or FACS report "FACS parameters."

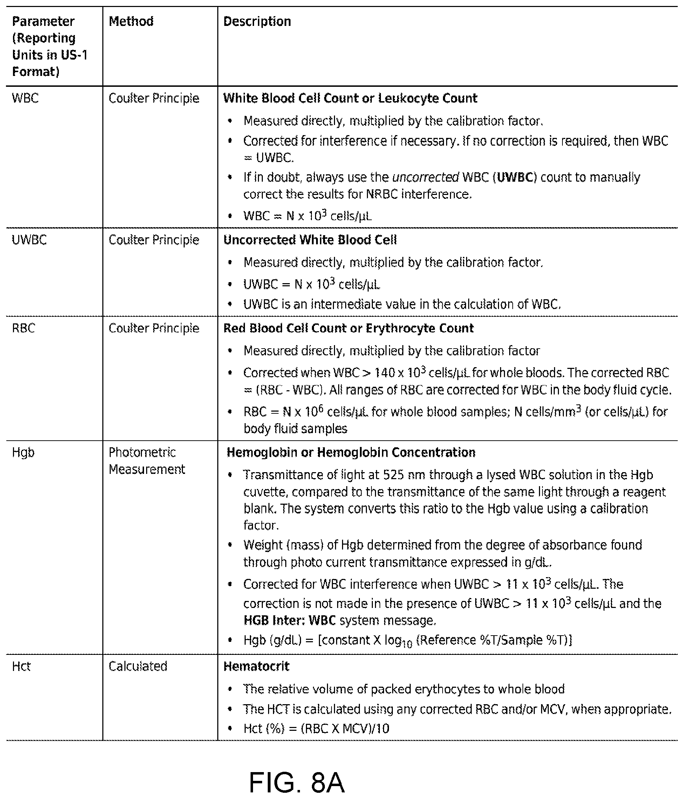

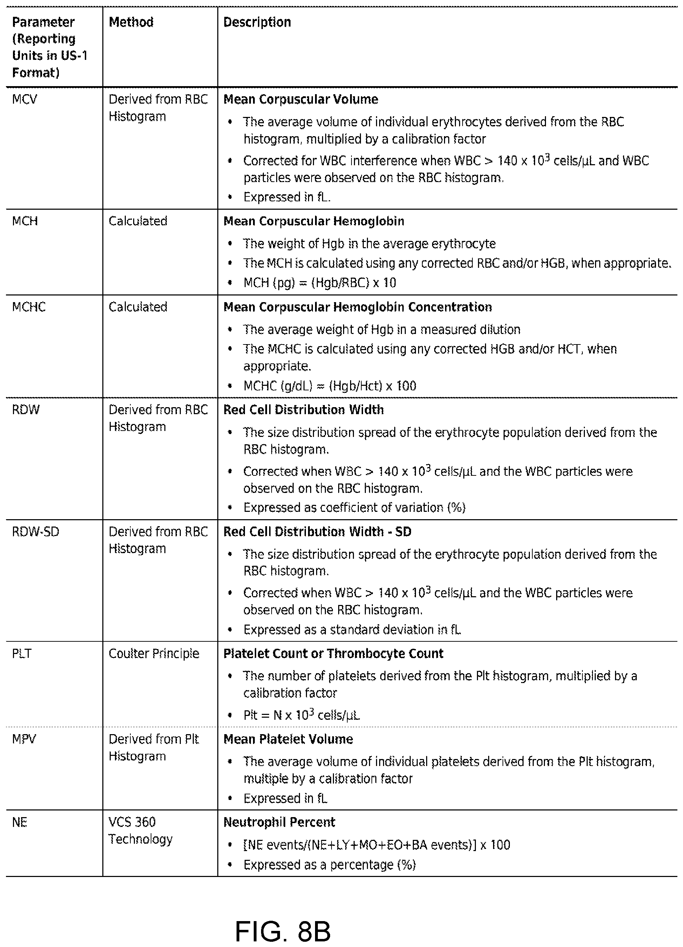

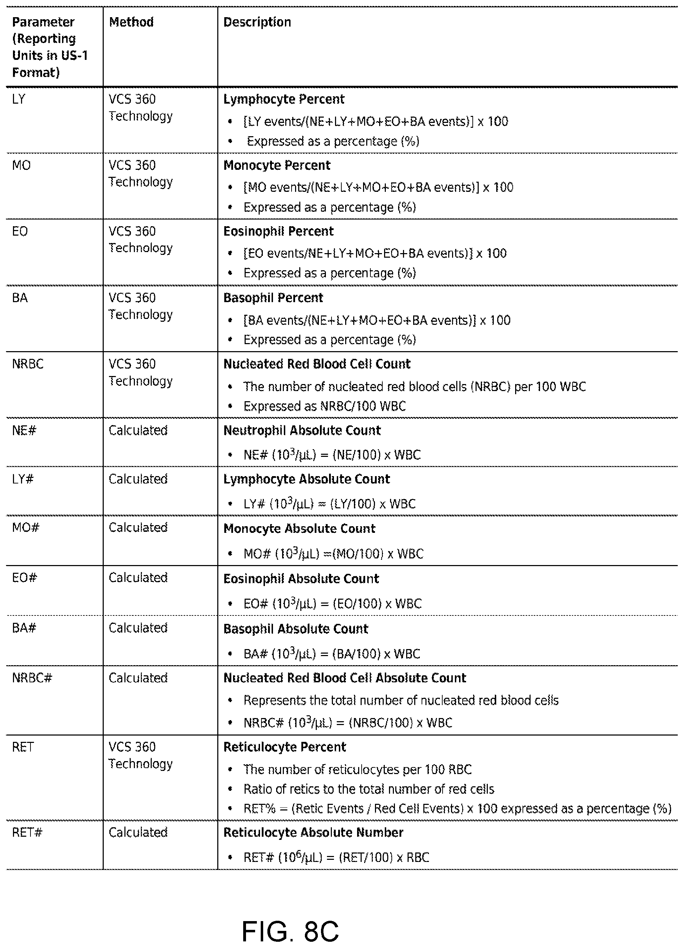

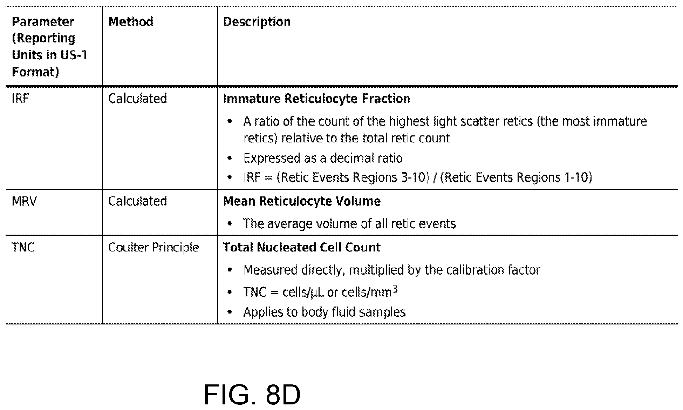

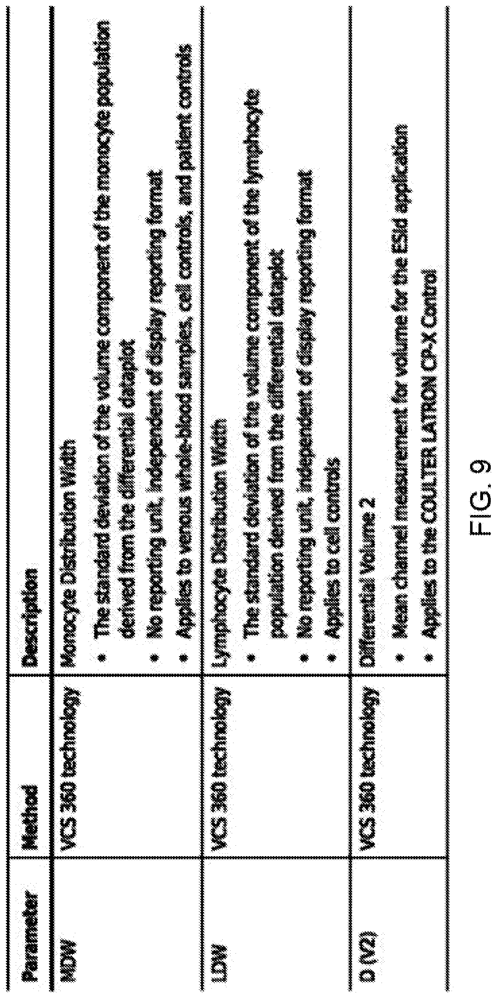

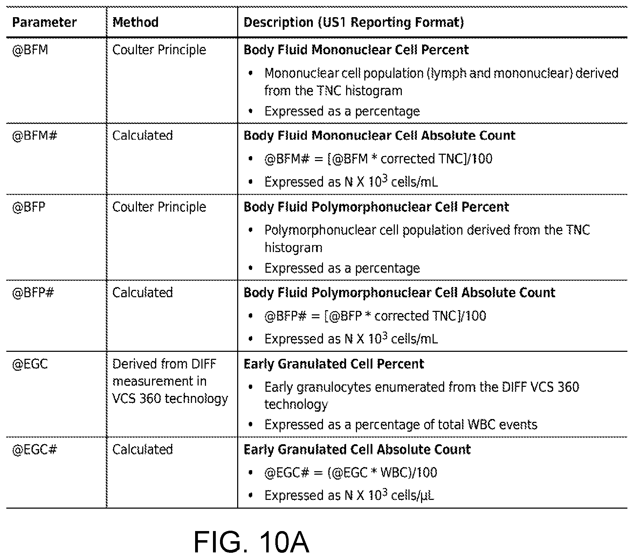

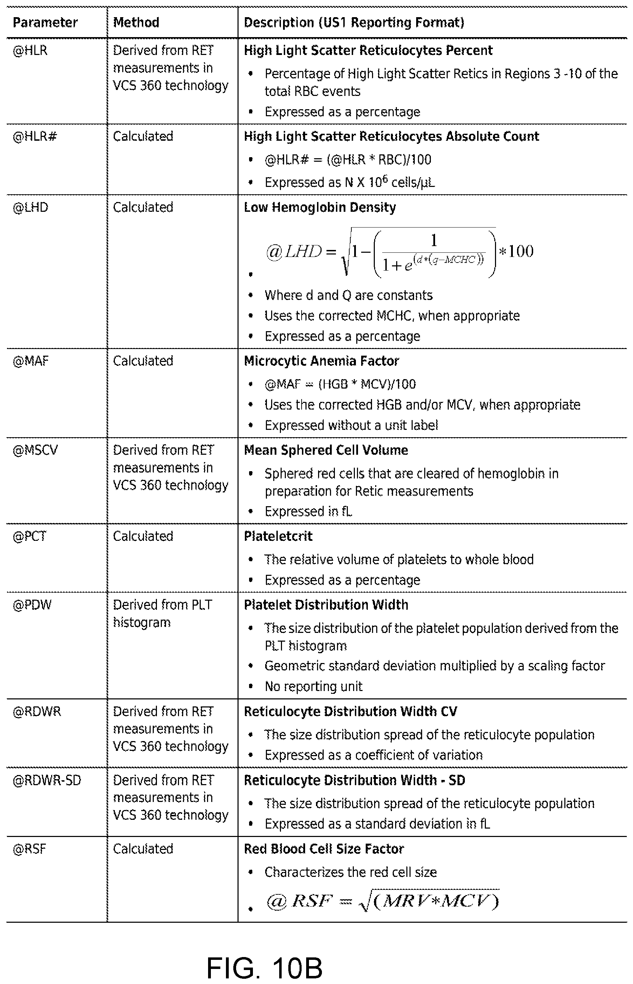

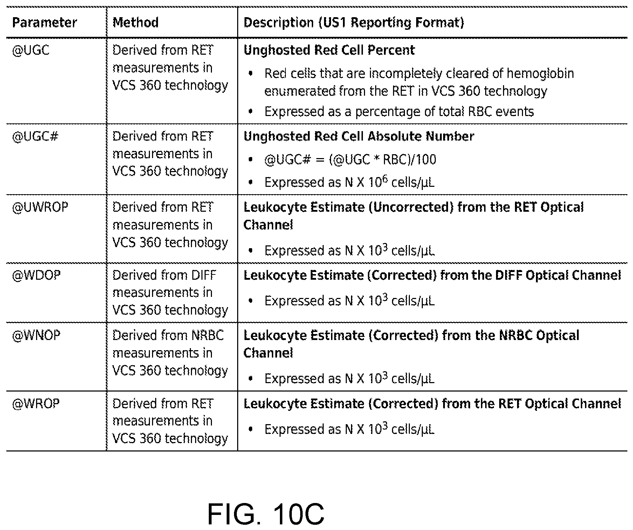

VCS parameters and FACS parameters are both described in the art. FIGS. 8A-8D, 9, and 10A-10C illustrate VCS parameters which may be used in methods or systems implemented based on this disclosure. Similarly, Table 1, below, illustrates FACS parameters which may be used in methods or systems implemented based on this disclosure.

**Table 1: FACS parameters**

| **Parameter** | **Description** |
|---|---|
| NEUT-GI, NEUT-RI | Neutrophil granularity and activation |
| HYPO-HE/HYPER-HE | Hypochromic and hyperchromic red blood cells |
| IPF# AND % | Immature Platelet Fraction |
| RET-HE | Reticulocyte haemoglobin equivalent |
| RE-LYMP, AS-LYMP | Reactive and antibody-synthesizing lymphocytes |
| NRBC | Nucleated red blood cells |
| IG COUNT | Immature granulocyte count |
| MICROR/MACROR | Microcytic and macrocytic red blood cells |
| HPC | Haematopoietic progenitor cells |
| FRC | Fragmented red blood cells |
| RE-MONO | Reactive monocytes |

In some methods described herein, the one or more cell population parameters may be VCS parameters or FACS parameters. In some methods, the cell population parameters may preferably be VCS parameters. In some methods, the cell population parameters may be VCS parameters selected from the group consisting of Ne_DC_Mean, Egc_Lmals_Mean, Egc_Mals_Mean (EGC), Ne_Lmals_Mean, Diff_LDW_Value, Ly_DC_SD, Ne_Mals_Mean, Rbc, Mo_DC_SD, MDW, Mo_DC_Mean, Ne_All_SD, Hct, Ne_DC_SD, Hgb, Ne_All_Mean, Ly_All_SD, NNrbc_Op_SD, Eo_All_Mean, Ly_Lmals_SD, Ne_Lals_SD, Mo_All_SD, Ly_Mals_SD, NRetic_Op_Mean, Eo_Lmals_Mean, Egc_All_Mean, Ly_Lals_SD, Ne_Lmals_SD, Ly_Op_SD, and combinations thereof.

In some aspects, the body fluid sample from a method such as described above may be whole blood. In some aspects, the one or more cell population parameters may comprise monocyte distribution width (MDW, also referred to as monocyte anisocytosis). MDW represents the volume distribution of the monocyte population. In some aspects, the one or more cell population parameters may be defined by a test order. In some aspects, the one or more cell population parameters may be compared to a threshold, such as a threshold value or a threshold range. In some aspects, a method such as described above may not subject the body fluid sample to dye prior to analysis. In some aspects, the MIS-C associated with SARS-CoV-2 may be suspected based on at least one cell population parameter, including, for example, MDW, being beyond a threshold value or a threshold range. In some aspects, in a method such as described above, the body fluid sample may be acquired from a hospitalized or non-hospitalized patient. In some aspects, in a method such as described above, the MIS-C associated with SARS-CoV-2 is evaluated automatically with a CBC differential test order.

### BRIEF DESCRIPTION OF THE DRAWINGS

FIG. 1 is a flowchart of an exemplary cellular analysis process in accordance with aspects of this disclosure.
FIG. 2 is a schematic depiction of an exemplary cellular analysis system in accordance with aspects of this disclosure.
FIG. 3 is an illustration of an exemplary transducer module and associated components in accordance with aspects of this disclosure.
FIG. 4 is a simplified block diagram of an exemplary module system in accordance with aspects of this disclosure.
FIG. 5 is a graph showing added value of MDW with EGC_MALS_MEAN.
FIG. 6 is a graph showing added value of MDW with NE_DC_SD.
FIG. 7 is a graph showing added value of MDW with EGC_LMALS_MEAN and NE_DC_SD.
FIGS. 8A-8D illustrate exemplary VCS parameters.
FIG. 9 illustrates exemplary VCS parameters.
FIGS. 10A-10C illustrate exemplary VCS parameters.

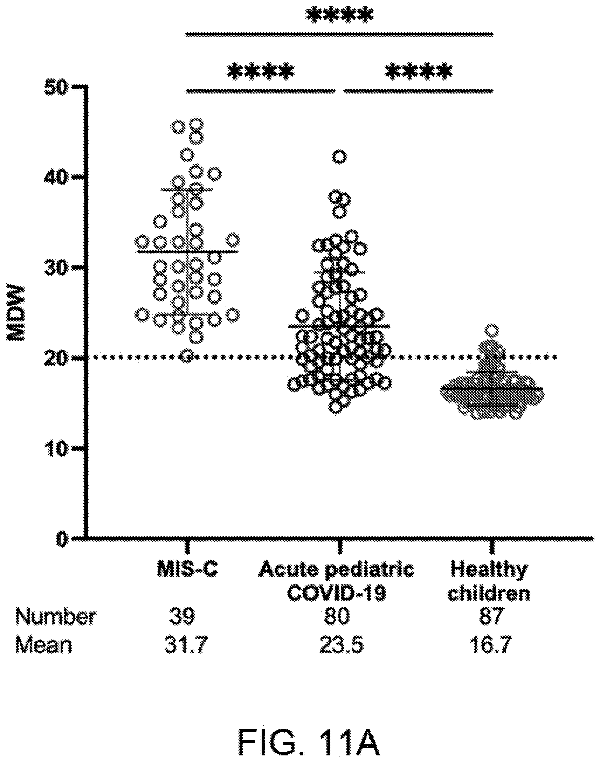

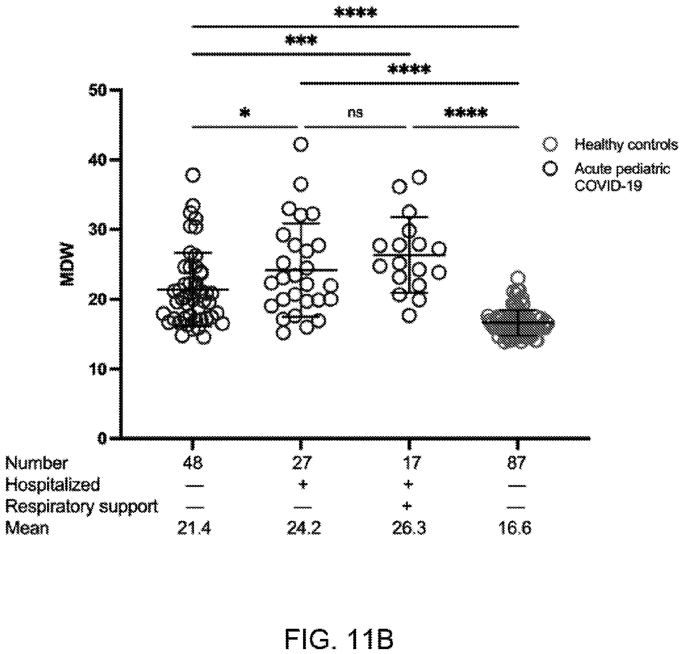

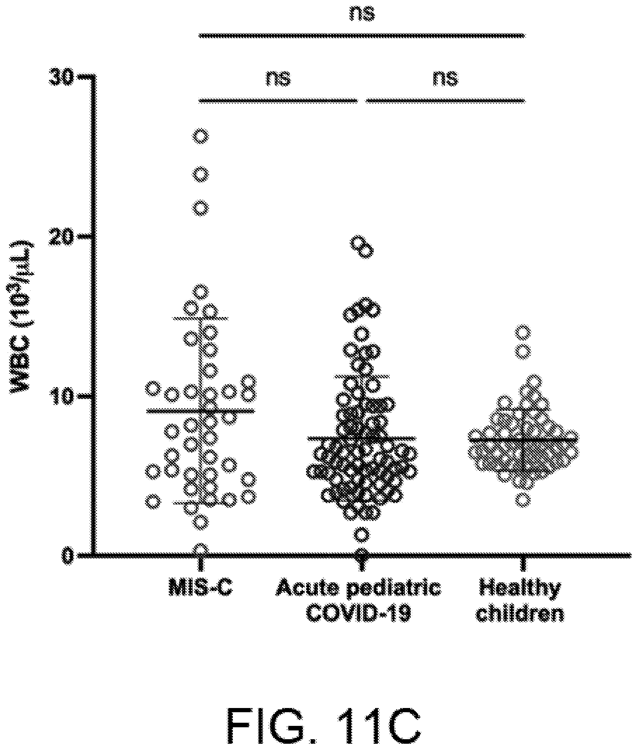

FIGS. 11A-11E illustrate MDW values for enrollees in a study evaluating parameters for MIS-C determination. FIG. 11A shows MDW values were significantly higher in MIS-C patients compared to acute COVID-19 infections. MDW values in both cases was higher than those in healthy controls. FIG. 11B shows among the patients with acute COVID-19, the highest MDW values were in the patients who were hospitalized and/or on respiratory support. FIG. 11C - FIG. 11E show that no significant differences in WBC, Lymphocytes, and monocyte numbers were observed between MIS-C, COVID-19, and healthy controls in the study group.

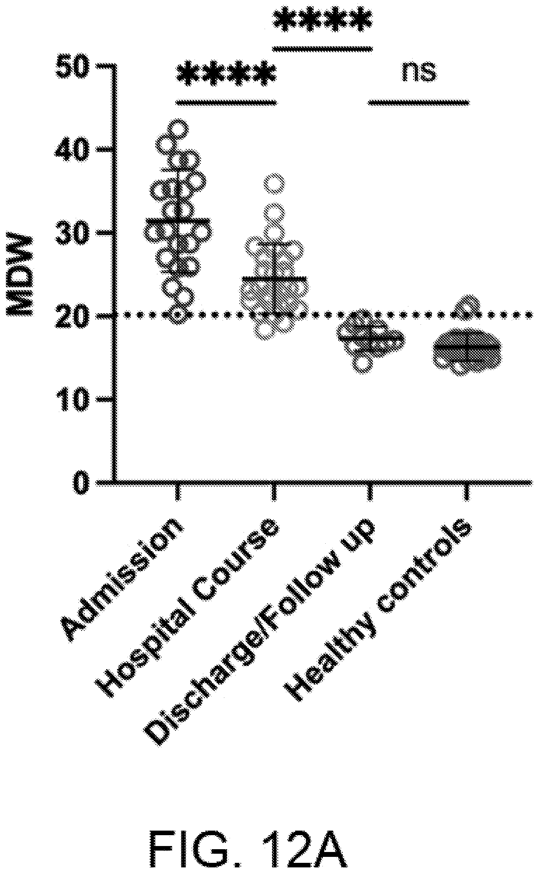

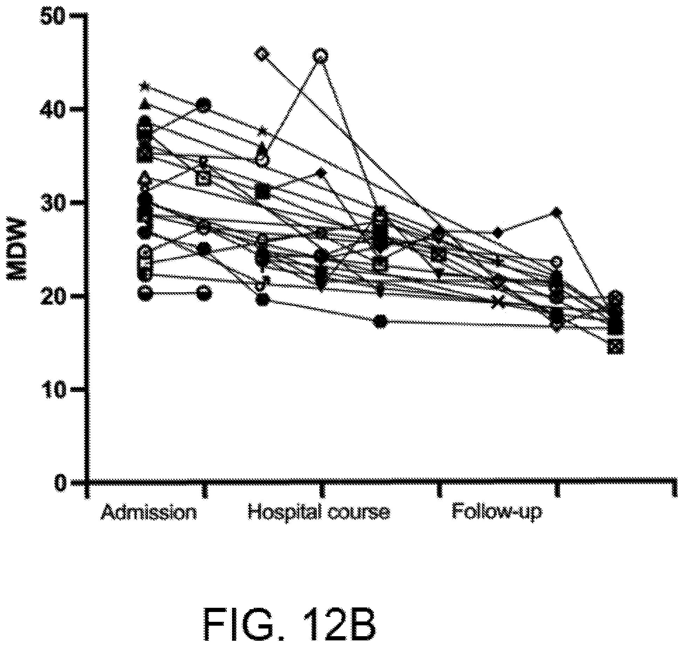

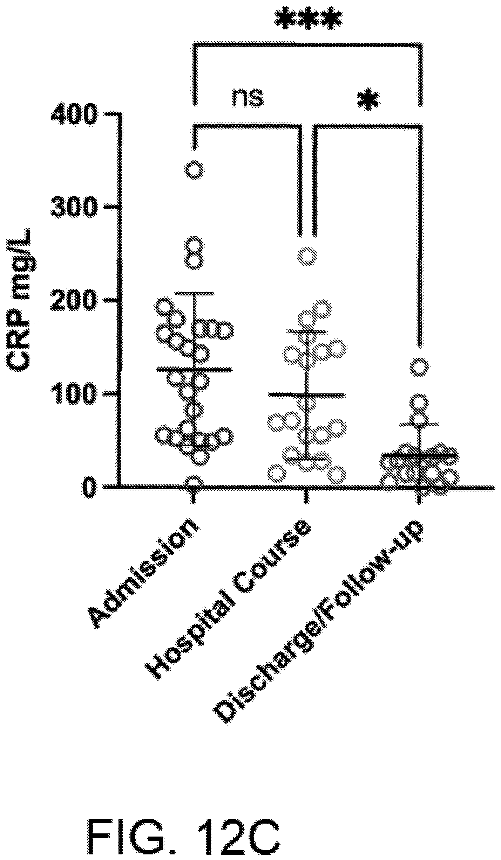

FIGS. 12A-12D illustrate parameter values for from admission through discharge of children enrolled in a study evaluating parameters for MIS-C determination. FIG. 12A shows MDW decreased between a first sample, taken prior to administration of MIS-C therapies (immunoglobulin or steroids), during hospital course, to the time of a second sample taken at discharge or in follow-up visits. MDW values showed persistent decrease during the hospital stay, from a mean of 32 at the time of admission to a mean of 17.60 at time of discharge/follow up, equivalent to MDW values of healthy controls. FIG. 12B shows MDW values from individual patients with MIS-C are plotted over course of their illness. FIG. 12C shows values of C-reactive protein (CRP), a generalized marker of inflammation, also decrease over course of a patient's illness, but CRP is slower to improve during the hospital stay. FIG. 12C shows values of WBC over course of a patient's illness; WBC did not reflect disease resolution across treatment course to follow up.
FIG. 13 illustrates a receiver operator curve for an MDW biomarker for MIS-C in a population of children with presenting with symptoms of infection.
FIGS. 14A-14B illustrate impact of age and sex on NMW. FIG. 14A shows the average MDW scores decreasing slightly with age. FIG. 14B shows the MDW values are comparable between male and female patients.

### DETAILED DESCRIPTION

As used herein, "patient" refers to a person or other animal from whom a body fluid sample is obtained, and may apply to research subjects, outpatients (people or animals seen for a brief visit with a medical practitioner, typically less than 1 day in duration, for medical assessment or treatment), inpatients (people or animals admitted to a medical care facility for 1 day or more, including, without limitation, hospitals, hospice care, rehabilitation facilities, and the like), or others. In some aspects, a patient may be under the current care of a clinician, such as a doctor, physician's assistant, nurse, nurse practitioner, chiropractor, surgeon, dentist, or the like. In some aspects, a "patient" may donate a sample without receiving medical assessment or treatment from the donation. In the methods of the invention, the patient is a pediatric patient. A pediatric patient may be up to 21 years of age. In some aspects, a pediatric patient may be at least 1 month of age and up to 21 years of age. In some aspects, a pediatric patient has an age of up to 21 years old, of up to 18 years old, and up to 16 years old. In some aspects, a pediatric patient has an age of up to one year old, up to two years old, and up to three years old.

Cellular analysis systems may use a variety of techniques to identify, count and/or characterize cells. For example, a cellular analysis system may use electrical impedance to determine the volume and quantity of cells passing through an interrogation zone in a flowcell. As another example, a cellular analysis system may use imaging technology to capture optical representations of the cell and analyze the optical representations (which might or might not be human-comprehensible or amenable to conversion to human-comprehensible images) to determine the size and quantity of cells in an interrogation zone, either in a quiescent system or in a flowcell. As yet another example, a cellular analysis system may use flow cytometry to irradiate cells passing through a flowcell and measure the transmission and/or scatter of the light as it passes through the cell. The light scatter may inherently distinguish different cells of different kinds, sizes or characteristics, or the cells may be prepared with markers, such as fluorescent markers, to facilitate the identification, quantification and/or characterization of the cells based on cellular features marked-or unmarked-by the marker.

A cellular analysis system may use combinations of these and/or other techniques to count, identify, and/or characterize cells. For example, a cellular analysis system may use a combination of electrical impedance and light scatter to analyze cells in a blood sample. If a combination of techniques is used, the techniques may employ hardware set up in serial progression (e.g., the same sample or aliquot of a sample is passed through multiple, separate interrogation zones), or in parallel progression (e.g., different aliquots of a sample are passed at essentially the same time through multiple, separate interrogation zones), or two or more techniques may be employed at the essentially the same time (e.g., a flow cell may be equipped to measure both electrical impedance and light scatter from the same sample or aliquot of sample in the same flow cell at essentially the same time). In this regard, essentially the same time means that the processes are running in overlapping time intervals for the same sample or different aliquots of the same sample. It is not essential to the practice of the invention that the different techniques be coordinated to occur at precisely the same time or in time intervals of equal duration.

A sample for analysis may be any biological fluid which contains cells. The biological fluid may, for example, be a body fluid sample such as blood. The sample may be whole blood, e.g., blood which has not been processed or modified except for the possible addition of an anticoagulant to prevent the blood from clotting, which would complicate flowing the blood through a flowcell for analysis. The sample may be processed, e.g., by dilution or by concentration. In some circumstances, the sample may be from a non-blood body fluid, such as urine, synovial fluid, saliva, bile, cerebrospinal fluid, amniotic fluid, semen, mucus, sputum, lymph, aqueous humour, tears, vaginal secretions, pleural fluid, pericardial fluid, peritoneal fluid, and the like. As with blood, if non-blood body fluids are sampled, the non-blood body fluids may be processed e.g., to achieve a desirable cellular concentration for analysis. A possible advantage of evaluating whole blood may be the relatively large number of cells available for analysis in a relatively small sample. A possible advantage of analyzing non-blood body fluids and/or processed blood may be pre-segregation of certain cells of interest and/or a reduction in the number of cells, because of differences in the types and number of cells that normally occur in different body fluids. A lower number of cells may be helpful, for example, for characterizing individual cells.

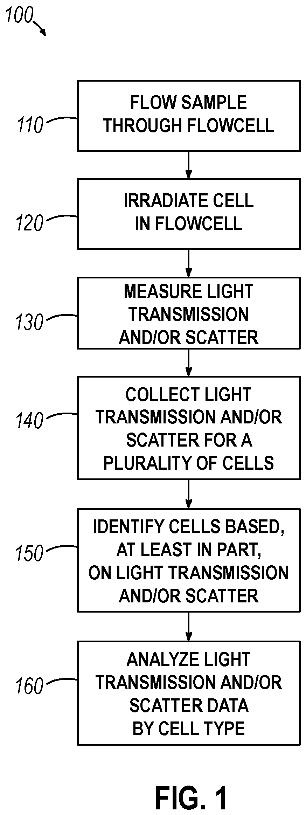

In some aspects, cells passing through a flowcell are analyzed using light scatter. As shown in FIG. 1, a method 100 for evaluating cell population variations may comprise flowing a sample through a flowcell 110. Cells within the sample flowing through the flowcell may be irradiated 120, as with visible light. The cellular analysis system may comprise one or more sensors which allow the analyzer to measure light transmission and/or scatter 130 as a cell is irradiated in the flowcell. The cellular analysis system may comprise a processor or means for communicating with a remote processor to collect the light transmission and/or scatter for a plurality of cells in the sample 140 as the cells flow through the flowcell. The processor may use an algorithm to identify cells based, at least in part, on light transmission and/or scatter 150. The processor, or a separate processor, may analyze the light transmission and/or scatter data for a particular cell or for a particular cell type population 160 (e.g., monocytes, neutrophils, red blood cells). The analysis could comprise, for example, calculating parameters, such as extrema, ranges, standard deviations, distribution widths, etc., for a particular measure, such as cell volume or light scatter, and/or for a particular cell type, such as monocytes, neutrophils, or red blood cells. For example, the cellular analysis system may calculate the standard deviation of light scatter, or of a particular angle of light scatter, such as upper median angle light scatter (UMALS), for cells identified as non-nucleated red blood cells (NNRBC). Additionally, cellular analysis system may calculate monocyte distribution width (MDW also referred to as monocyte anisocytosis), a calculation of the standard deviation of cell volumes within the subpopulation of monocytes within a blood sample. In some examples, measurement 130 could involve alternative measurements of cell size and/or granularity, such as image analysis, electrical impedance, radiofrequency (RF) response, flow cytometry with or without markers, alone or in combination or sub-combinations, and with or without light transmission and/or light scatter measures.

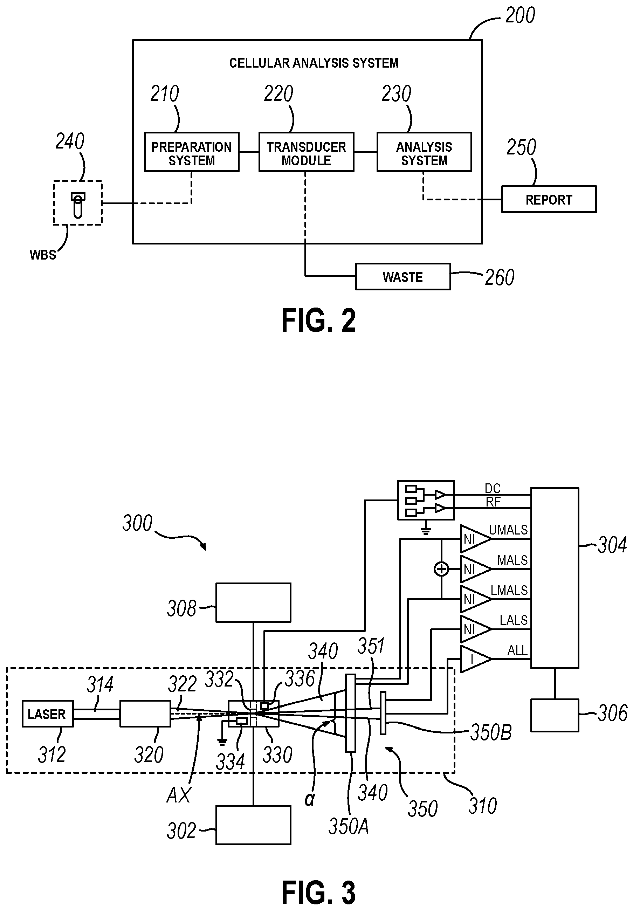

FIG. 2 schematically depicts a cellular analysis system 200. As shown here, system 200 includes a preparation system 210, a transducer module 220, and an analysis system 230. While system 200 is described generally with reference to three core system blocks (210, 220, and 230), the skilled artisan readily understands that system 200 may include other system components such as central control processor(s), display system(s), fluidic system(s), temperature control system(s), user-safety control system(s), and the like. In operation, a whole blood sample (WBS) 240 can be presented to the system 200 for analysis. In some instances, WBS 240 is aspirated into system 200. Exemplary aspiration techniques are known to the skilled artisan.

After aspiration, WBS 240 can be delivered to a preparation system 210. Preparation system 210 receives WBS 240 and can perform operations involved with preparing WBS 240 for further measurement and analysis. For example, preparation system 210 may separate WBS 240 into one or more predefined aliquots for presentation to transducer module 220. In some aspects, preparation system 210 may make no changes to the composition of WBS 240. Alternately, preparation system 210 may include mixing chambers so that appropriate reagents may be added to one or more of the aliquots. For example, where an aliquot is to be tested for differentiation of white blood cell subset populations, a lysing reagent (e.g., ERYTHROLYSE, a red blood cell lysing buffer) may be added to the aliquot to break up and remove the RBCs. Preparation system 210 may also include temperature control components to control the temperature of the reagents and/or mixing chambers. Appropriate temperature controls can improve the consistency of the operations of preparation system 210, and may facilitate pre-treatment of cells in the sample, e.g., with fluorescent markers, stains, or lyse.

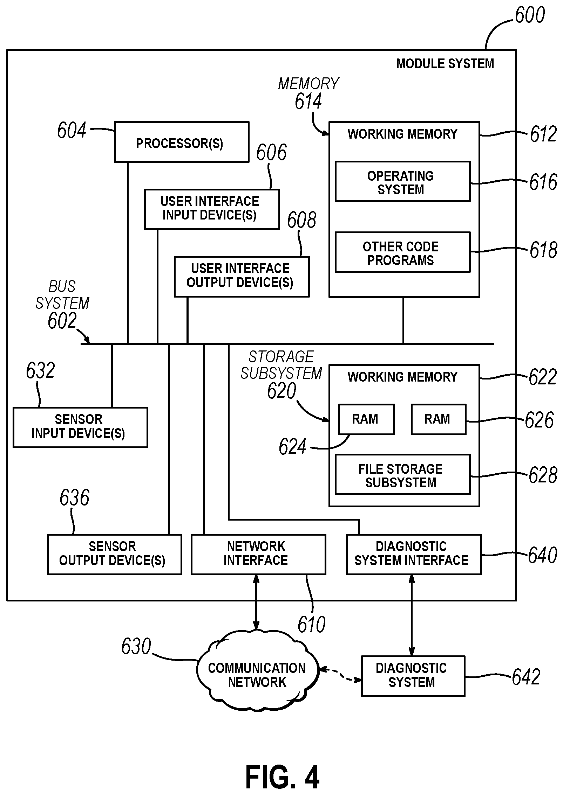

In some instances, one or more predefined aliquots can be transferred from preparation system 210 to transducer module 220. As described in further detail below, transducer module 220 can perform light transmission, and/or light scatter measurements of cells from the WBS 240 passing individually therethrough. Measured light propagation (e.g., light transmission, light scatter) parameters can be provided or transmitted to analysis system 230 for data processing. In some instances, analysis system 230 may include computer processing features and/or one or more modules or components such as those described herein with reference to the system depicted in FIG. 4 and described further below, which can evaluate the measured parameters, identify and enumerate at least one of the blood cellular constituents, and calculate cell population parameters for one or more cell populations within the aliquot.

As shown in FIG. 2, cellular analysis system 200 may generate or output a report 250 containing measurements and/or calculated parameters for one or more cell populations within the aliquot, e.g., monocyte volume distribution width, neutrophil volume distribution width, a count or percentage of immature granulocytes, and/or a standard deviation of a UMALS measurement for NNRBC. In some instances, excess biological sample from transducer module 220 can be directed to an external (or, alternatively, internal) waste system 260. An exemplary cellular analysis system is a Beckman Coulter DxH hematology analyzer, which measures direct current impedance to determine cell volume, as well as conductivity and light scatter, for cytoplasmic granularity and nuclear structure.

FIG. 3 illustrates in more detail a transducer module and associated components in more detail. As shown here, system 300 includes a transducer module 310 having a light or irradiation source such as a laser 312 emitting a beam 314. The laser 312 can be, for example, a 635 nm, 5 mW, solid-state laser. In some instances, system 300 may include a focus-alignment system 320 that adjusts beam 314 such that a resulting beam 322 is focused and positioned at a cell interrogation zone 332 of a flow cell 330. In some instances, the flow cell 330 receives a sample aliquot from a preparation system 302. Various fluidic mechanisms and techniques can be employed for hydrodynamic focusing of the sample aliquot within flow cell 330.

In some instances, the aliquot generally flows through the cell interrogation zone 332 such that its constituents pass through the cell interrogation zone 332 one at a time. In some cases, a system 300 may include a cell interrogation zone or other feature of a transducer module or blood analysis instrument such as those described in U.S. Pat. Nos. 5,125,737; 6,228,652; 7,390,662; 8,094,299; 8,189,187; and 9,939,453. For example, a cell interrogation zone 332 may be defined by a square transverse cross-section measuring approximately 50×50 microns, and having a length (measured in the direction of flow) of approximately 65 microns. Flow cell 330 may include an electrode assembly having first and second electrodes 334, 336 for performing DC impedance and/or RF conductivity measurements of the cells passing through cell interrogation zone 332.

Signals from electrodes 334, 336 can be transmitted to the analysis system 304. The electrode assembly can analyze volume and conductivity characteristics of the cells using low-frequency current and high-frequency current, respectively. For example, low-frequency DC impedance measurements can be used to analyze the volume of each individual cell passing through the cell interrogation zone. High-frequency RF current measurements can be used to determine the conductivity of cells passing through the cell interrogation zone. Because cell walls act as conductors to high frequency current, the high frequency current can be used to detect differences in the insulating properties of the cell components, as the current passes through the cell walls and through each cell interior. High frequency current can be used to characterize nuclear and granular constituents and the chemical composition of the cell interior.

The light source in FIG. 3 has been described as a laser, however, the light source may alternatively or additionally include a xenon lamp, an LED lamp, an incandescent lamp, or any other suitable source of light, including combinations of the same or different kinds of lamps (e.g., multiple LED lamps or at least one LED lamp and at least one xenon lamp). As shown in FIG. 3, for example, incoming beam 322 irradiates the cells passing through cell interrogation zone 332, resulting in light propagation within an angular range α (e.g. scatter, transmission) emanating from the zone 332. Exemplary systems are equipped with sensor assemblies that can detect light within one, two, three, four, five, or more angular ranges within the angular range α, including light associated with an extinction or axial light loss measure.

As shown in FIG. 3, light propagation 340 can be detected by a light detection assembly 350, optionally having a light scatter detector unit 350A and a light scatter and/or transmission detector unit 350B. In some instances, light scatter detector unit 350A includes a photoactive region or sensor zone for detecting and measuring upper median angle light scatter (UMALS), for example, light that is scattered or otherwise propagated at angles relative to a light beam axis within a range from about 20 to about 42 degrees. In some instances, UMALS corresponds to light propagated within an angular range from between about 20 to about 43 degrees, relative to the incoming beam axis which irradiates cells flowing through the interrogation zone. Light scatter detector unit 350A may also include a photoactive region or sensor zone for detecting and measuring lower median angle light scatter (LMALS), for example, light that is scattered or otherwise propagated at angles relative to a light beam axis within a range from about 10 to about 20 degrees. In some instances, LMALS corresponds to light propagated within an angular range from between about 9 to about 19 degrees, relative to the incoming beam axis which irradiates cells flowing through the interrogation zone.

A combination of UMALS and LMALS is defined as median angle light scatter (MALS), which may be light scatter or propagation at angles between about 9 degrees and about 43 degrees relative to the incoming beam axis which irradiates cells flowing through the interrogation zone. One of skill in the art will understand that these angles (and the other angles described herein) may vary somewhat based on the configuration of the interrogation, sensing and analysis systems.

As shown in FIG. 3, the light scatter detector unit 350A may include an opening 351 that allows low angle light scatter or propagation 340 to pass beyond light scatter detector unit 350A and thereby reach and be detected by light scatter and transmission detector unit 350B. According to some embodiments, light scatter and transmission detector unit 350B may include a photoactive region or sensor zone for detecting and measuring lower angle light scatter (LALS), for example, light that is scattered or propagated at angles relative to an irradiating light beam axis of less than about 5.1 degrees. In some instances, LALS corresponds to light propagated at an angle of less than about 9 degrees, relative to the incoming beam axis which irradiates cells flowing through the interrogation zone. In some instances, LALS corresponds to light propagated at an angle of less than about 10 degrees, relative to the incoming beam axis which irradiates cells flowing through the interrogation zone. In some instances, LALS corresponds to light propagated at an angle of about 1.9 degrees±0.5 degrees, relative to the incoming beam axis which irradiates cells flowing through the interrogation zone. In some instances, LALS corresponds to light propagated at an angle of about 3.0 degrees±0.5 degrees, relative to the incoming beam axis which irradiates cells flowing through the interrogation zone. In some instances, LALS corresponds to light propagated at an angle of about 3.7 degrees±0.5 degrees, relative to the incoming beam axis which irradiates cells flowing through the interrogation zone. In some instances, LALS corresponds to light propagated at an angle of about 5.1 degrees±0.5 degrees, relative to the incoming beam axis which irradiates cells flowing through the interrogation zone. In some instances, LALS corresponds to light propagated at an angle of about 7.0 degrees±0.5 degrees, relative to the incoming beam axis which irradiates cells flowing through the interrogation zone. In each instance, LALS may correspond to light propagated an angle of about 1.0 degrees or more. That is, LALs may correspond to light propagated at angles between about 1.0 degrees and about 1.9 degrees; between about 1.0 degrees and about 3.0 degrees; between about 1.0 degrees and about 3.7 degrees; between about 1.0 degrees and about 5.1 degrees, between about 1.0 degrees and about 7.0 degrees, between about 1.0 degrees and about 9.0 degrees; or between about 1.0 degrees and about 10.0 degrees.

According to some embodiments, light scatter and transmission detector unit 350B may include a photoactive region or sensor zone for detecting and measuring light transmitted axially through the cells, or propagated from the irradiated cells, at an angle of about 0 degrees relative to the incoming light beam axis. In some cases, the photoactive region or sensor zone may detect and measure light propagated axially from cells at angles of less than about 1 degree relative to the incoming light beam axis. In some cases, the photoactive region or sensor zone may detect and measure light propagated axially from cells at angles of less than about 0.5 degrees relative to the incoming light beam axis less. Such axially transmitted or propagated light measurements correspond to axial light loss (ALL or a derivative thereof, e.g., AL2). As noted in U.S. Pat. No. 7,390,662, when light interacts with a particle, some of the incident light changes direction through the scattering process (i.e., light scatter) and part of the light is absorbed by the particles. Both of these processes remove energy from the incident beam. When viewed along the incident axis of the beam, the light loss can be referred to as forward extinction or axial light loss. Additional aspects of axial light loss measurement techniques are described in U.S. Pat. No. 7,390,662 at column 5, line 58 to column 6, line 4.

As such, the cellular analysis system 300 provides means for obtaining light propagation measurements, including light scatter and/or light transmission, for light emanating from the irradiated cells of the biological sample at any of a variety of angles or within any of a variety of angular ranges, including ALL and multiple distinct light scatter or propagation angles. For example, light detection assembly 350, including appropriate circuitry and/or processing units, provides a means for detecting and measuring UMALS, LMALS, LALS, MALS, and ALL.

Wires or other transmission or connectivity mechanisms can transmit signals from the electrode assembly (e.g. electrodes 334, 336), light scatter detector unit 350A, and/or light scatter and transmission detector unit 350B to the analysis system 304 for processing. For example, measured DC impedance, RF conductivity, light transmission, and/or light scatter parameters can be provided or transmitted to the analysis system 304 for data processing. In some instances, analysis system 304 may include computer processing features and/or one or more modules or components such as those described herein with reference to the system depicted in FIG. 4, which can evaluate the measured parameters, identify and enumerate biological sample constituents, and correlate a subset of data characterizing elements of the biological sample with one or more features or parameters of interest.

As shown in FIG. 3, cellular analysis system 300 may generate or output a report 306 presenting measurements made or parameters calculated for the sample, such as WBC, MDW, or UMALS mean for NNRBC. In some instances, excess biological sample from transducer module 310 can be directed to an external (or alternatively internal) waste system 308. In some instances, a cellular analysis system 300 may include one or more features of a transducer module or blood analysis instrument such as those described in U.S. Pat. Nos. 5,125,737; 6,228,652; 8,094,299; 8,189,187 and 9,939,453.

FIG. 4 is a simplified block diagram of an exemplary module system that broadly illustrates how individual system elements for a module system 600 may be implemented in a separated or more integrated manner. Module system 600 may be part of or in connectivity with a cellular analysis system 200. Module system 600 is well suited for producing data or receiving input related to cellular analysis. In some instances, module system 600 includes hardware elements that are electrically coupled via a bus subsystem 602, including one or more processors 604, one or more input devices 606 such as user interface input devices, and/or one or more output devices 608 such as user interface output devices. In some instances, system 600 includes a network interface 610, and/or a diagnostic system interface 640 that can receive signals from and/or transmit signals to a diagnostic system 642. In some instances, system 600 includes software elements, for example, shown here as being currently located within a working memory 612 of a memory 614, an operating system 616, and/or other code 618, such as a program configured to implement one or more aspects of the techniques disclosed herein. Memory 614 may be non-transitory and/or embodied in tangible media, such as hardware.

Module system 600 may include a storage subsystem 620 that can store the basic programming and data constructs that provide the functionality of the various techniques disclosed herein. For example, software modules implementing the functionality of method aspects, as described herein, may be stored in storage subsystem 620. These software modules may be executed by the one or more processors 604. In a distributed environment, the software modules may be stored on a plurality of computer systems and executed by processors of the plurality of computer systems. Storage subsystem 620 can include memory subsystem 622 and file storage subsystem 628. Memory subsystem 622 may include a number of memories including a main random access memory (RAM) 626 for storage of instructions and data during program execution and a read only memory (ROM) 624 in which fixed instructions are stored. File storage subsystem 628 can provide persistent (non-volatile) storage for program and data files, and may include tangible storage media which may optionally embody patient, treatment, assessment, or other data. File storage subsystem 628 may include a hard disk drive, a floppy disk drive along with associated removable media, a Compact Digital Read Only Memory (CD-ROM) drive, an optical drive, DVD, CD-R, CD RW, solid-state removable memory, other removable media cartridges or disks, and the like. One or more or all of the drives may be located at remote locations on other connected computers at other sites coupled to module system 600. In some instances, systems may include a computer-readable storage medium or other tangible storage medium that stores one or more sequences of instructions which, when executed by one or more processors, can cause the one or more processors to perform any aspect of the techniques or methods disclosed herein. One or more modules implementing the functionality of the techniques disclosed herein may be stored by file storage subsystem 628. In some instances, the software or code may provide protocol to allow the module system 600 to communicate with communication network 630. Optionally, such communications may include dial-up or internet connection communications.

System 600 can be configured to carry out various aspects of methods of the present disclosure. For example, processor component or module 604 can be a microprocessor control module configured to receive cellular parameter signals from a sensor input device or module 632, from a user interface input device or module 606, and/or from a diagnostic system 642, optionally via a diagnostic system interface 640 and/or a network interface 610 and a communication network 630. In some instances, sensor input device(s) may include or be part of a cellular analysis system that is equipped to obtain multiple light angle detection parameters, such as in a Beckman Coulter DxH^{™} hematology analyzer. In some instances, user interface input device(s) 606 and/or network interface 610 may be configured to receive cellular parameter signals generated by a cellular analysis system that is equipped to obtain multiple light angle detection parameters, such as a Beckman Coulter DxH^{™} Hematology Analyzer. In some instances, diagnostic system 642 may include or be part of a cellular analysis system that is equipped to obtain multiple light angle detection parameters, such as a Beckman Coulter DxH^{™} Hematology Analyzer.

Processor component or module 604 can also be configured to transmit cellular parameter signals, optionally processed according to any of the techniques disclosed herein or known to one of skill in the art, to sensor output device or module 636, to user interface output device or module 608, to network interface device or module 610, to diagnostic system interface 640, or any combination thereof. Each of the devices or modules of the present disclosure can include one or more software modules on a computer readable medium that is processed by a processor, or hardware modules, or any combination thereof. Any of a variety of commonly used platforms, such as Windows, MacIntosh, and Unix, along with any of a variety of commonly used programming languages, may be used to implement embodiments of the present disclosure.

User interface input devices 606 may include, for example, a touchpad, a keyboard, pointing devices such as a mouse, a trackball, a graphics tablet, a scanner, a joystick, a touchscreen incorporated into a display, audio input devices such as voice recognition systems, microphones, and other types of input devices. User input devices 606 may also download a computer executable code from a tangible storage media or from communication network 630, the code embodying any of the methods or aspects thereof disclosed herein. It will be appreciated that terminal software may be updated from time to time and downloaded to the terminal as appropriate. In general, use of the term "input device" is intended to include a variety of conventional and proprietary devices and ways to input information into module system 600.

User interface output devices 606 may include, for example, a display subsystem, a printer, a fax machine, or non-visual displays such as audio output devices. The display subsystem may be a cathode ray tube (CRT), a flat-panel device such as a liquid crystal display (LCD), a projection device, or the like. The display subsystem may also provide a non-visual display such as via audio output devices. In general, use of the term "output device" is intended to include a variety of conventional and proprietary devices and ways to output information from module system 600 to a user. In some instances, a cellular analysis system may not directly include a user interface output device, instead transferring data to a network, computer processor, or computer-readable non-transitory storage medium, with data display for a human user occurring in connection with that device or with devices to which the data from the cellular analysis system is further transferred after the initial transfer. If data is transferred from the analyzer without display, the data transferred may be raw sensor data or processed data or a combination of raw and processed data.

Bus subsystem 602 provides a mechanism for letting the various components and subsystems of module system 600 communicate with each other as intended or desired. The various subsystems and components of module system 600 need not be at the same physical location but may be distributed at various locations within a distributed network. Although bus subsystem 602 is shown schematically as a single bus, alternate examples of the bus subsystem may utilize multiple busses.

Network interface 610 can provide an interface to an outside network 630 or other devices. Outside communication network 630 can be configured to effect communications as needed or desired with other systems. It can thus receive an electronic packet from module system 600 and transmit any information as needed or desired back to module system 600. As depicted here, communication network 630 and/or diagnostic system interface 642 may transmit information to or receive information from a diagnostic system 642 that is equipped to obtain multiple light angle detection parameters, such as such as a Beckman Coulter DxH ^{™} Cellular Analysis System. As non-limiting examples, outside communication network 630 may be used to transmit data between a cellular analysis system and a research database, a laboratory information system (LIS), an electronic medical record (EMR), and the like. In some instances, the communication may be one-way, with information flowing from the cellular analysis system to other systems. In some instances, the communication may be one-way with information (such as orders for specific measurements to be made or population parameters to be calculated) flowing from an external system, which may be remote or physically proximate to the cellular analysis system, to the cellular analysis system. In some instances, the communication may be two-way. In some instances, the information communicated to the cellular analysis system by an external system may include patient information useful in evaluating the significance of cellular measurements. For example, some reference ranges for hematology parameters may differ for pediatric populations or specific patient sub-populations relative to a general adult population, and the cellular analysis system may consider patient information when determining whether to flag analytical results for further review.

In addition to providing such infrastructure communications links internal to the system, the communications network system 630 may also provide a connection to other networks such as the internet and may comprise a wired, wireless, modem, and/or other type of interfacing connection.

It will be apparent to the skilled artisan that substantial variations may be used in accordance with specific requirements. For example, customized hardware might also be used and/or particular elements might be implemented in hardware, software (including portable software, such as applets), firmware, or combinations thereof. Further, connection to other computing devices such as network input/output devices may be employed. Module terminal system 600 itself can be of varying types including a computer terminal, a personal computer, a portable computer, a workstation, a network computer, or any other data processing system. Due to the ever-changing nature of computers and networks, the description of module system 600 depicted in FIG. 4 is intended only as a specific example for purposes of illustrating one or more examples of the present disclosure. Many other configurations of module system 600 are possible having more or less components than the module system depicted in FIG. 4. Any of the modules or components of module system 600, or any combinations of such modules or components, can be coupled with, or integrated into, or otherwise configured to be in connectivity with, any of the cellular analysis system embodiments disclosed herein. Relatedly, any of the hardware and software components discussed above can be integrated with or configured to interface with other medical assessment or treatment systems used at other locations.

In some instances, the module system 600 can be configured to receive one or more cellular analysis parameters of a patient at an input module. Cellular analysis parameter data can be transmitted to an assessment module where raw sensor data or partially analyzed sensor data is further processed and/or evaluated in conjunction with additional information, possibly including prior laboratory results for the same patient; laboratory results from other types of analyzers or laboratory analyses; non-laboratory data about the patient, such as patient complaints, diagnostic history, vitals, or physical examination findings, or combinations thereof. The cellular analysis including a cell population parameter, such as WBC, MDW, NNRBC UMALS mean, and other cell population parameters can be output to a system user via an output module.

Analysis system 304 of transducer module 300 or other code programs 618 of module system 600 or both may comprise one or more algorithms for processing sensor data generated by transducer module 300. The one or more algorithms may process the sensor data to identify and count cells. For example, individual cells may be identifiable based on light scatter and/or light transmission data that provides an indication of the size and certain surface properties of the cells. As another example, electrical impedance may provide an indication of the size of the cell. Radiofrequency conductivity may provide an indication of cellular constituents that may be useful in distinguishing granulocytes and nucleated versus non-nucleated cells. Markers, stains, image analysis or other measurement techniques may be used to identify cells as, for example, NNRBC, WBC, monocytes, neutrophils, and the like. An algorithm may count the number of signals consistent with a given cell type during an interrogation period, which may be defined by time through a flowcell or imaging time, or may be defined by a volume of body fluid examined, or both.

In some instances, the signals produced are continuous or ordinal values, and the magnitude or other properties of the signals may be further analyzed. For example, higher electrical impedance values typically indicate a larger cell, and may be useful for identifying a particular cell. Electrical impedance values may also correlate to cell volume, and therefore the magnitude of the signals across many cells in the sample may also convey useful information about that sub-population of cells. For example, electrical impedance values may help identify monocytes and distribution features of the volumes for the monocytes as a sub-population of cells may convey information about MIS-C status.

Using a cellular analysis system such as described above, hematology markers may be identified and used in distinguishing hospitalized SARS-CoV-2 positive and MIS-C from outpatient SARS-CoV-2 positive and negative cases and hospitalized SARS-CoV-2 negative. Multiple studies assessing the effectiveness of such parameters have been performed, two of which are discussed below.

In a first study, particular markers and combinations of markers were identified in a study of data collected from 86 pediatric (1 to 18 years) patients, who presented to the emergency department. In the data, there were 16 hospitalized SARS-CoV-2 cases and 70 non-hospitalized SARS-CoV-2 cases. Of 16 hospitalized cases, 13 were MIS-C and 3 were Hospitalized-COVID positive; and of 70 non-hospitalized cases, 8 hospitalized COVID negative, 23 outpatient COVID positive, and 39 outpatient COVID negative. This data was used to statistically analyze the efficacy of 190 markers in determining MIS-C status. Specifically, for each marker, area under curve (AUC) was calculated using one predictor logistic regression model. Additionally, the cutoff and corresponding sensitivity, specificity, PPV and NPV were calculated using Youden's J statistic, a statistic that captures the performance of a dichotomous diagnostic test. The top 29 markers with an AUC of at least 80% which were identified in this analysis are presented below in Table 2.

**Table 2: Markers found in a first study to have AUC of at least 0.80 in determining MIS-C.**

| **Markers** | **Cell Type or Characteristic definition** | **AUC** | **Youden cutoff** | **Sensitivity** | **Specificity** | **PPV** | **NPV** |
|---|---|---|---|---|---|---|---|
| Ne_DC_Mean | Neutrophil | 0.96 | 151.80 | 0.94 | 0.89 | 0.65 | 0.98 |
| Egc_Lmals_Mean | Early Granulated Cell | 0.95 | 135.08 | 0.80 | 0.93 | 0.80 | 0.93 |
| Egc_Mals_Mean (EGC) | Early Granulated Cell | 0.95 | 145.90 | 0.87 | 0.90 | 0.76 | 0.95 |
| Ne_Lmals_Mean | Neutrophil | 0.94 | 136.87 | 0.94 | 0.90 | 0.68 | 0.98 |
| Diff_LDW_Value | Lymphocyte Distribution Width | 0.94 | 13.96 | 0.94 | 0.76 | 0.47 | 0.98 |
| Ly_DC_SD | Lymphocyte | 0.93 | 16.78 | 0.81 | 0.89 | 0.62 | 0.95 |
| Ne_Mals_Mean | Neutrophil | 0.93 | 141.96 | 0.94 | 0.80 | 0.52 | 0.98 |
| Rbc | Red blood cell | 0.92 | 4.60 | 0.94 | 0.71 | 0.43 | 0.98 |
| Mo_DC_SD | Monocyte | 0.92 | 22.42 | 0.81 | 0.89 | 0.62 | 0.95 |
| MDW | Monocyte Distribution Width | 0.91 | 22.31 | 0.81 | 0.89 | 0.62 | 0.95 |
| Mo_DC_Mean | Monocyte | 0.91 | 175.48 | 0.75 | 0.91 | 0.67 | 0.94 |
| Ne_All_SD | Neutrophil | 0.91 | 11.20 | 0.88 | 0.80 | 0.50 | 0.97 |
| Hct | Hematocrit | 0.91 | 33.85 | 0.69 | 0.93 | 0.69 | 0.93 |
| Ne_DC_SD | Neutrophil | 0.91 | 18.44 | 0.88 | 0.83 | 0.54 | 0.97 |
| Hgb | Hemoglobin | 0.90 | 12.45 | 0.81 | 0.76 | 0.43 | 0.95 |
| Ne All Mean | Neutrophil | 0.90 | 149.51 | 0.81 | 0.94 | 0.76 | 0.96 |
| Ly_All_SD | Lymphocyte | 0.89 | 11.95 | 0.75 | 0.91 | 0.67 | 0.94 |
| NNrbc_Op_SD | Non-Nucleated Red Blood Cell The degree of variation in all populations of WBC (Ly, Mo, Ne, Eo, Ba) | 0.89 | 38.94 | 0.63 | 0.97 | 0.83 | 0.92 |
| Eo All Mean | Eosinophil | 0.87 | 124.58 | 0.75 | 0.84 | 0.52 | 0.94 |
| Ly_Lmals_SD | Lymphocyte | 0.86 | 21.77 | 0.56 | 0.96 | 0.75 | 0.91 |
| Ne_Lals_SD | Neutrophil | 0.85 | 33.08 | 0.88 | 0.73 | 0.42 | 0.96 |
| Mo All SD | Monocyte | 0.85 | 13.59 | 0.81 | 0.73 | 0.41 | 0.94 |
| Ly_Mals_SD | Lymphocyte | 0.84 | 16.95 | 0.88 | 0.64 | 0.36 | 0.96 |
| NRetic_Op_Mean | All mature RBC's (not retics) | 0.83 | 69.51 | 0.88 | 0.66 | 0.37 | 0.96 |
| Eo Lmals Mean | Eosinophils | 0.83 | 187.01 | 0.69 | 0.80 | 0.44 | 0.92 |
| Egc_All_Mean | Early Granulated Cell | 0.83 | 159.50 | 0.60 | 0.90 | 0.69 | 0.86 |
| Ly_Lals_SD | Lymphocyte | 0.83 | 12.77 | 0.69 | 0.84 | 0.50 | 0.92 |
| Ne_Lmals_SD | Neutrophil | 0.80 | 12.52 | 0.88 | 0.66 | 0.37 | 0.96 |
| Ly_Op_SD | Lymphocyte | 0.80 | 10.18 | 0.56 | 0.93 | 0.64 | 0.90 |

In the table above (Table 2), and elsewhere in this document, the initial portion of the marker designation (e.g., in column 1 of Table 2), with the exception of the markers beginning with "Diff_" refers to the cell type or characteristic identified in column 2 of Table 2. Further, in the table above (Table 2), and elsewhere in this document, references to DC should be understood as referring to volume, references to OP or Op should be understood as referring to opacity, references to UMALS should be understood as referring to upper median angle light scatter, references to LMALS or Lmals should be understood as referring to lower median angle light scatter, references to MALS or Mals should be understood as referring to median angle light scatter (e.g., weighted sum of UMALS and LMALS), references to ALL or All should be understood as referring to axial light loss, references to AL2 should be understood as referring to a derivative of ALL which has been scaled and calibrated to compensate for manufacturing variation in optical components, references to LALS or lals should be understood as referring to low angle light scatter, references to DW should be understood as referring to distribution width (including, for example, monocyte distribution width (MDW) and lymphocyte distribution width (LDW)), references to SD should be understood as referring to standard deviation, and references to Mean should be understood as referring to the mean. For example, NRetic_Op_Mean should be understood as referring to the mean opacity for all mature red blood cells (i.e., those that are not retics). In another example, Mo_DC_SD should be understood as referring to the standard deviation in the volume of the monocytes. In Table 2 and elsewhere in this document, Diff_LDW_Value should be understood as the value reported for the lymphocyte distribution width (LDW). In some implementations, this may be used as a control as part of the MDW parameter, with lymphocyte distribution width (LDW, also referred to as Diff_LDW_Value). Rbc should be understood as red blood cell count, as further described in FIG. 8A. Hct should be understood as hematocrit, as further described in FIG. 8A. Hgb should be understood as hemoglobin, as further described in FIG. 8A.

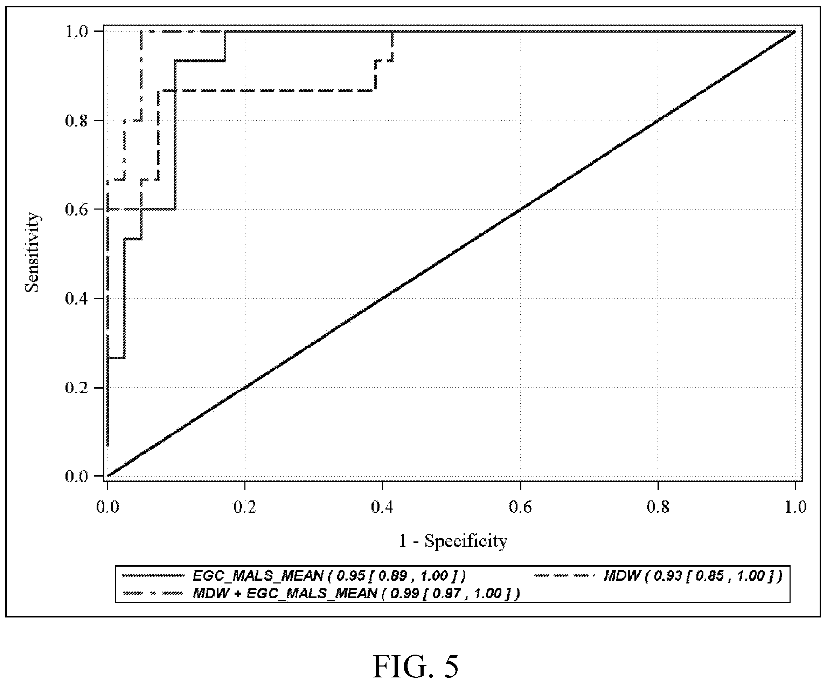

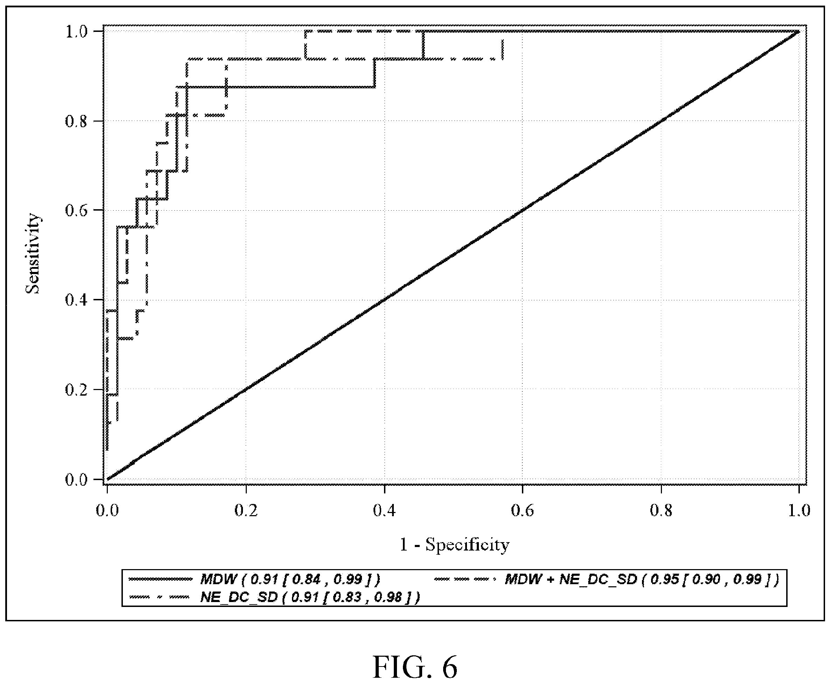

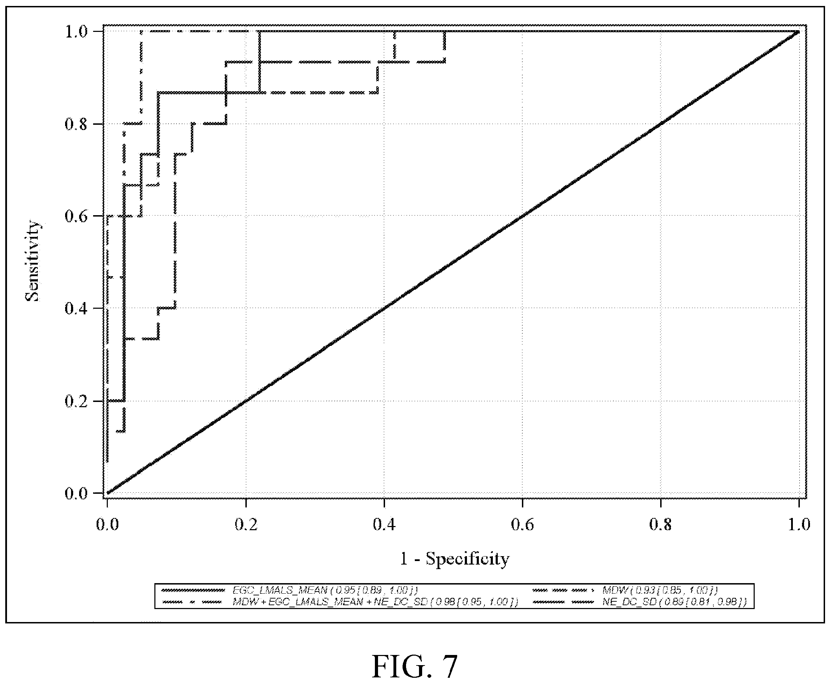

In addition to analyzing individual markers using the calculations described above, an added value analysis was also conducted to determine the performance of MDW in conjunction with other markers. As shown in FIG. 5, this added value analysis indicated that MDW in combination with the mean of the median angle light scatter of early granulated cells (Egc_Mals_Mean or EGC) can improve diagnostic ability to provide an AUC of approximately 99%. Additionally, as shown in FIGS. 6-7, other combinations may also provide improved results including, for example, better positive predictive value, negative predictive value, or increased AUC, relative to a single monocyte or granulocyte cell population parameter alone. For example, as shown in FIG. 6, MDW in combination with the standard deviation in the volume of neutrophils (Ne_DC_SD) provided an AUC of 95%; as shown in FIG. 7, MDW in combination with the standard deviation in the volume of neutrophils (Ne_DC_SD) and the mean of the lower median angle light scatter of early granulated cells (ECG_LMALS_MEAN) provided an AUC of 98%. It is believed that the use of any of the non-MDW parameters set forth in Table 2 in combination with MDW will facilitate accurate SARS-CoV-2 associated MIS-C detection. The efficacy of MDW as a standalone parameter was also evaluated and, as shown in Table 2, MDW was found to be effective even when not combined with other parameters.

In a second study, 699 children, ages 21 years and younger, presenting to a hospital with persistent fever were enrolled, and 822 blood samples were collected for analysis by a hematology analyzer. Of the 699 children, 424 children presented with symptoms concerning for infection, of whom 46 met the criteria for MIS-C and 94 of whom had acute COVID-19. Non-infectious (n=193) and healthy (n=90) controls were also enrolled. The average age of the participants was 10 years (range 4 days - 21 years). Sex was equally distributed (52% male, 48% female). By race, participants were white (n=363, 52%), black (n=148), Asian (n=26), American Indian (n=3) and Hispanic (n-217). In this study, it was found that children with MIS-C displayed a significantly higher MDW (mean 31.7) than children with other infections, non-infectious illnesses, and healthy controls (mean MDW 23.8, 19.5, and 16.7, respectively). Differences between multiple groups were analyzed using the one-way-ANOVA parametric test with Tukey's posthoc test. Differences were considered statistically significant for p<0.05. Dependencies between blood parameters were calculated using Pearson's correlation coefficients, with correlations assumed to be significant for p<0.05. A Receiver Operator Curve (ROC) was calculated for the ability of MDW in distinguishing MIS-C from children presenting with other infections.

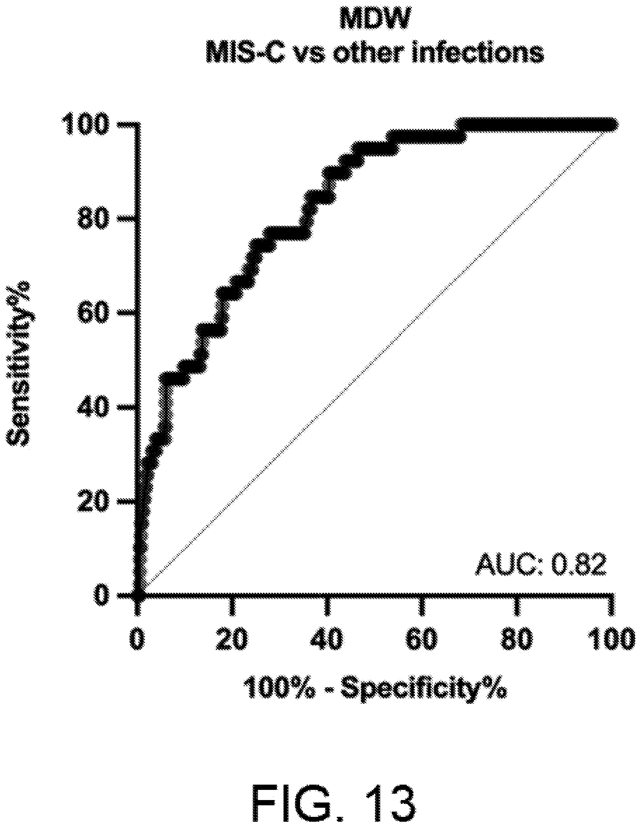

In this study, receiver-operating classification (ROC) analysis revealed that a MDW biomarker would have an 82% diagnostic accuracy identifying children with MIS-C in a population of children presenting with symptoms of infection. As such, when a child presents with non-specific symptoms of infection in the setting of high community rates of COVID-19, this study indicated that, as shown in FIG. 13, an MDW greater than 31 would have a 90% specificity in identifying MIS-C. Alternatively, in this same clinical situation, an MDW below 23 would have a 95% sensitivity. Overall, in children with symptoms of infection, MDW values above 31 would strongly suggest MIS-C, while MDW values below 23 would aid in exclusion of MIS-C from the differential diagnosis.

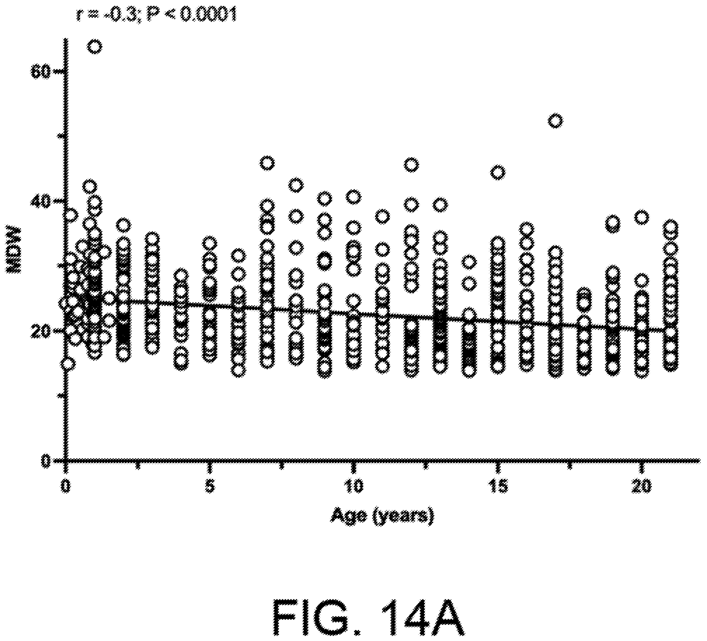

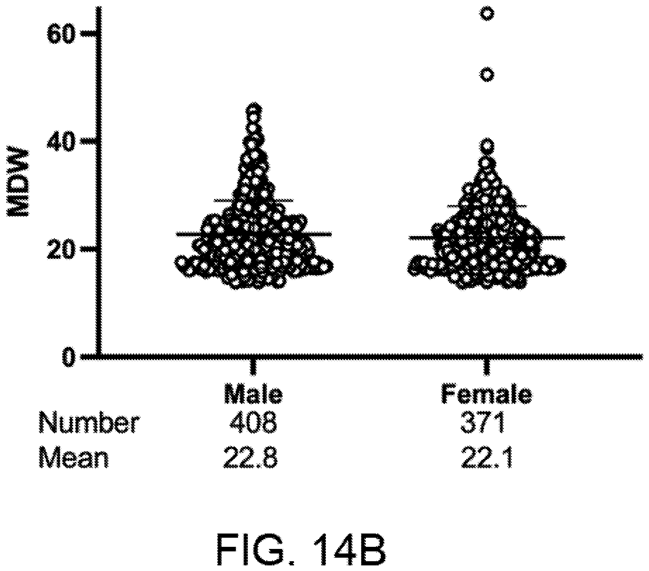

The second study further sought to assess whether age would impact MDW values across the cohort, regardless of diagnosis or symptoms. Consistent with previous studies reporting MDW values are slightly higher in younger children compared to older children and young adults, MDW was found to be increased in younger children), with children <2 years of age having the highest MDW (mean 25.3) and children >16 years of age having the lowest MDW (mean 20.1). This can be seen in FIGS. 14A and 14B.

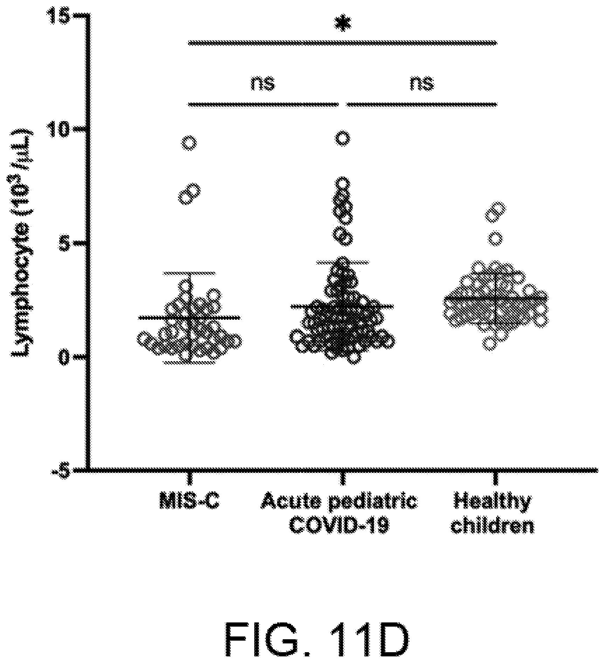

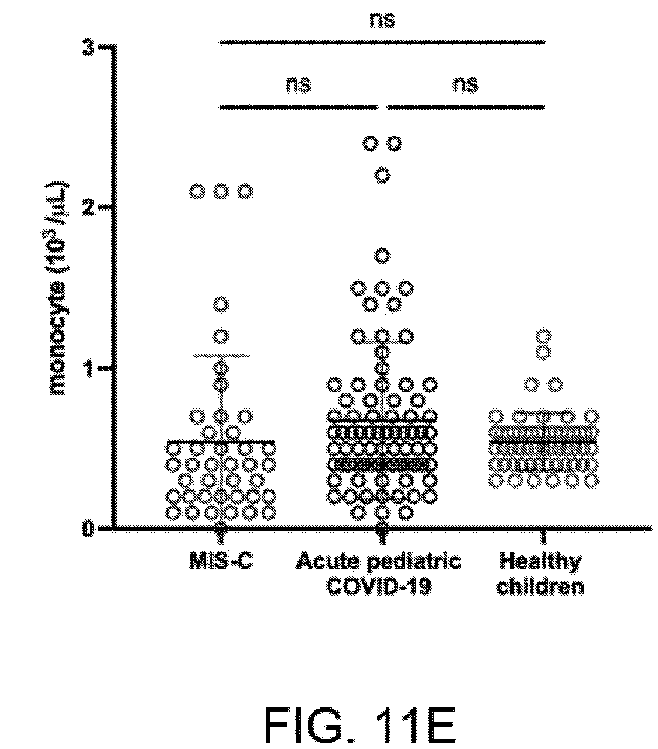

This second study also sought to assess whether MDW would distinguish MIS-C from acute pediatric SARS-CoV-2 infection. The study found that MDW was significantly elevated in children with MIS-C as compared to children with acute COVID-19. This result can be seen in FIG. 11A (acute COVID-19 mean 23.5, one way ANOVA, P<0.0001). Additionally, MDW was found to be increased in mild pediatric COVID-19 compared to healthy controls, and higher MDW values were noted in children with COVID-19 requiring hospitalization. This can be seen in FIG. 11B (one way ANOVA, P<0.0001), suggesting MDW may serve as a disease severity marker for COVID-19-related illness. In this study, no differences were noted when comparing mean white blood cell count or mean monocyte count from children with MIS-C with children with acute COVID-19, or healthy controls, and children with MIS-C only showed a mild lymphopenia. This can be seen in FIGS. 11C, 11D and 11E.

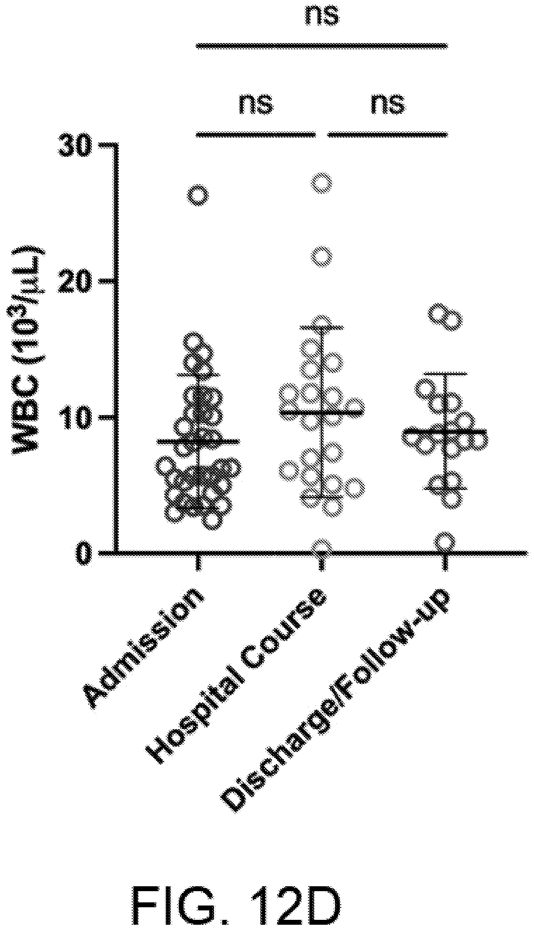

This study further indicated that, not only was MDW useful in identifying MIS-C, it could also aid in tracking disease resolution. In the study, MDW was measured at multiple time points in children with MIS-C and individual values were plotted over time: prior to administration of MIS-C therapies (immunoglobulin or steroids) or within 24 hours of admission, during hospital course, and at follow-up. This can be seen in FIG. 12A. MDW dropped significantly during the hospital stay, from a mean of 32 at time of admission to a mean of 17.60 at time of discharge/follow up, equivalent to MDW values of healthy controls reflecting the expected resolution of illness. This can be seen in FIG. 12B. These findings corresponded with generalized markers of inflammation (C-reactive protein: CRP), although CRP is slower to improve during the hospital course, as shown in FIG. 12C. Importantly, typical parameters on the CBC did not reflect clinical trajectory; for example, WBC did not reflect disease resolution across treatment course to follow up, as shown in FIG. 12D.

Beyond MDW, this second study also indicated that additional cell population parameters, as shown in Table 3 could also be useful in diagnosing and/or tracking the progress of MIS-C.

**Table 3: Markers found in a second study to have AUC of at least 0.80 in determining MIS-C.**

| **Marker** | **AUC** | **StdErr** | **LowerArea** | **UpperArea** |
|---|---|---|---|---|
| Mo_DC_SD | 0.9328 | 0.0216 | 0.8905 | 0.975 |
| MDW | 0.9253 | 0.0233 | 0.8796 | 0.9709 |
| Egc_Lmals_Mean | 0.898 | 0.0458 | 0.8082 | 0.9878 |
| Ly_DC_SD | 0.8767 | 0.0347 | 0.8087 | 0.9448 |
| Diff_LDW_Value | 0.8711 | 0.0355 | 0.8016 | 0.9407 |
| Ne_All_SD | 0.865 | 0.033 | 0.8002 | 0.9298 |
| Mo_All_SD | 0.8556 | 0.04 | 0.7772 | 0.9339 |
| Hct | 0.8484 | 0.0349 | 0.7799 | 0.9169 |
| Ly_All_SD | 0.8339 | 0.0393 | 0.757 | 0.9109 |
| Ne_DC_SD | 0.8295 | 0.0389 | 0.7532 | 0.9059 |
| Ne_Lmals_Mean | 0.8259 | 0.0403 | 0.7468 | 0.9049 |
| Egc_Op_Mean | 0.8228 | 0.0526 | 0.7197 | 0.9258 |
| Mo_DC_Mean | 0.8223 | 0.0462 | 0.7318 | 0.9129 |
| NNrbc_Op_SD | 0.821 | 0.0394 | 0.7437 | 0.8982 |
| Ne_Lals_SD | 0.8144 | 0.0418 | 0.7325 | 0.8963 |
| Hgb | 0.8136 | 0.0402 | 0.735 | 0.8923 |
| NrbcAL2Mean | 0.8122 | 0.0452 | 0.7237 | 0.9008 |
| Egc_Umals_SD | 0.8065 | 0.0544 | 0.6998 | 0.9132 |

As noted previously in the context of the first study, combinations of markers may also be used to provide further power for detecting MIS-C. For example, the combinations of markers set forth in table 4 were found to have an AUC of at least 0.99, and the combinations of markers set forth in table 5 were found to have an AUC of at least 0.95.

**Table 4: Exemplary combinations of three markers for MIS-C detection.**

| **Marker Combination** | **AUC** | **StdErr** | **LowerArea** | **UpperArea** |
|---|---|---|---|---|
| Diff_LDW_Value + Egc_Op_Mean + NrbcAL2Mean | 0.9979 | 0.00258 | 0.9928 | 1 |
| Diff_MDW_Value + Egc_Umals_SD + NrbcAL2Mean | 0.9956 | 0.00432 | 0.9871 | 1 |
| Egc_Op_Mean + Ly_All_SD + NrbcAL2Mean | 0.9947 | 0.00485 | 0.9852 | 1 |
| Egc_Op_Mean + Ly_DC_SD + NrbcAL2Mean | 0.9947 | 0.00508 | 0.9847 | 1 |
| Egc_Umals_SD + Mo_DC_SD + NrbcAL2Mean | 0.9923 | 0.00639 | 0.9798 | 1 |
| MDW + Egc_Op_Mean + NrbcAL2Mean | 0.9912 | 0.0074 | 0.9767 | 1 |
| Egc_Op_Mean + Mo_DC_SD + NrbcAL2Mean | 0.9912 | 0.0074 | 0.9767 | 1 |

**Table 5: Exemplary combinations of two markers for MIS-C detection**

| **Marker Combination** | **Area** | **StdErr** | **LowerArea** | **UpperArea** |
|---|---|---|---|---|
| MDW + | 0.9812 | 0.0145 | 0.9528 | 1 |
| Egc_Umals_SD | | | | |
| MDW + Egc_Op_Mean | 0.9762 | 0.0169 | 0.9431 | 1 |
| Egc_Umals_SD + Mo_DC_SD | 0.9742 | 0.0165 | 0.9419 | 1 |
| MDW + Egc_Lmals_Mean | 0.9732 | 0.0177 | 0.9386 | 1 |
| Egc_Op_Mean + Mo_DC_SD | 0.9702 | 0.0196 | 0.9318 | 1 |
| Egc_Lmals_Mean + Mo_DC_SD | 0.9683 | 0.019 | 0.931 | 1 |
| Mo_DC_SD + NrbcAL2Mean | 0.9576 | 0.0173 | 0.9237 | 0.9916 |
| MDW + NrbcAL2Mean | 0.9563 | 0.0174 | 0.9222 | 0.9903 |

In general, it is believed that combinations of parameters (e.g., parameters from Tables 2 and/or 3) related to different types of cells may provide information which could be more effective in detecting MIS-C than information from any of the individual parameters. Thus, a combination which includes one parameter from two or more of the following groups: monocyte parameters (e.g., MDW), granulocyte parameters (e.g., Ne_DC_SD, Egc_Lmals_Mean), red blood cell parameters (e.g., Hgb), lymphocyte parameters (e.g., Ly_All_SD), and eosinophil parameters (e.g., Eo_All_Mean), is expected to be useful in detecting MIS-C. Additionally, it is believed that combinations of parameters related to monocytes, granulocytes (e.g., neutrophils), and red blood cells are likely to be useful in detecting MIS-C, as monocytes, granulocytes, and red blood cells share a common progenitor cell in bone marrow. Additionally, lymphocyte parameters would also be expected to be relevant in such combinations, and so a four parameter combination such as MDW + Ne_DC_Mean + Rbc + Ly_All_SD would be expected to be suitable for use in detecting MIS-C, though other combinations (e.g., combinations with fewer parameters, such as shown in Tables 4 and 5) are also possible, and so the above description should be understood as being illustrative only, and should not be treated as limiting.

As further described above, various parameters and combinations of parameters may be used in addressing (including, for example, identifying or treating) MIS-C. For example, a system may be implemented to, based on measurements of a cell population parameter (including, for example, those shown in Table 2 or Table 3) either individually or in combination, rapidly detect and report SARS-CoV-2 associated MIS-C. In another example, a method may include detecting MIS-C or detecting the severity of MIS-C in a patient, based at least in part on a measurement of a cell population parameter, either alone or in combination. The MIS-C may preferably be SARS-CoV-2 associated MIS-C. In some aspects, the cell population parameter preferable includes MDW, either alone or in combination with one more additional cell population parameters. In some aspects, the method of detecting MIS-C or detecting the severity of MIS-C in a patient may further include assessing an additional parameter recommended for assessing MIS-C such as complete blood count (CBC), C-reactive protein (CRP), erythrocyte sedimentation rate (ESR), fibrinogen, procalcitonin, d-dimer, ferritin, lactic acid dehydrogenase (LDH), interleukin 6 (IL-6), and albumin.

In some aspects, if one of the cell population parameters described herein or a combination of those cell population parameters is greater than a threshold value, this could be treated as indicating MIS-C. A threshold value may be determined by a person having skill in the art for the specific patient population being examined. In some aspects, the threshold value may include a Youden cutoffs set forth in Table 2. In some aspects, including, for example when the patient is a pediatric patient, the threshold value for MDW may be at least 23, or at least 24. In some aspects, the threshold value for MDW may be at least 24, a value indicating MIS-C, as described in the second study. In some aspects, including, for example when the patient is a pediatric patient, the threshold value for MDW may be up to 31. In some aspects, the threshold value for MDW may be age-adjusted. For example, when the patient is a pediatric patient of up to 1 year of age, up to 2 years of age, or up to 3 years of age, the threshold value or threshold range for MDW may be age-adjusted.

In some aspects, cell population parameters including those shown in Table 2 or Table 3 may be used in determining timing and/or administration of treatment for MIS-C cases. For example, MDW values may be used as an aid in determining risk for certain complications associated with MIS-C and/or for selecting patients for whom particular therapies are appropriate. An appropriate therapy may be selected by the patient's physician depending on the MDW value; exemplary therapies might include extracorporeal membrane oxygenation (ECMO), intravenous immunoglobulin (IVIG), steroids, and vasopressors. In another example, MDW values, and in particular declining MDW values, may be used in evaluating efficacy of treatment. When, for example, treatment is determined to be efficacious based on declining MDW values, a clinician may be able to more accurately determine when a hospitalized MIS-C cases may be appropriately de-escalated and/or discharged. A threshold value for determining when a patient may be de-escalated and/or discharged may be determined by a person having skill in the art for the specific patient population being examined. For example, a threshold value for determining when a patient may be de-escalated and/or discharged may be determined by the mean value of the cell population parameter in a normal population. In some aspects, including, for example when the patient is a pediatric patient, the threshold value of MDW for de-escalation and/or discharge may be up to 20, up to 21, up to 22, up to 23, or up to 24. For example, in some aspects, the threshold value of MDW for de-escalation and/or discharge may be up to 20, as shown in FIG. 12A. In some aspects, the threshold value of MDW for de-escalation and/or discharge may be up to 24, a value indicating MIS-C, as described in the second study. In some aspects, the threshold value for MDW for when a patient may be de-escalated and/or discharged may be age-adjusted. For example, when the patient is a pediatric patient of up to 1 year of age, up to 2 years of age, or up to 3 years of age, the threshold value or threshold range for MDW may be age-adjusted.

One of skill in the art will understand that the methods, systems and parameters described are not intended to exhaustively describe all possible implementations of the disclosed technology. For example, while the disclosed techniques may be implemented using systems which perform analysis on samples of blood in a near-native state, the described approach of determining MIS-C using hematology parameters may also be useful for analyzers which use fluorescent staining or similar treatment on their samples. Similarly, the described approach of determining MIS-C using hematology parameters may also be useful when performed using other parameters, such as parameters which, like MDW, may reflect variation in immune cell population. Exemplary other parameters include the NEMO score, described in Urrechaga, et. al., Role of Leucocytes Cell Population Data in Early Detection of Sepsis, J. CLIN. PATHOL. 2017; 71:259-266; or an immature granulocyte (IG) count, including one obtained as described in Nigro et al., Performance of an automated immature granulocyte count as a predictor of neonatal sepsis, AM J CLIN PATH. 123: 618-624, 2005. Similarly, the thresholds set forth herein for any given cell population parameter may vary based on the methods used to measure or observe the cells, as well as the specific hardware (e.g., light source, sensing hardware) used to make the measurements.

The dimensions and values disclosed herein are not to be understood as being strictly limited to the exact numerical values recited. Instead, unless otherwise specified, each such dimension is intended to mean both the recited value and a functionally equivalent range surrounding that value to include at least the variability due to the reproducibility of measurements made using the test methods described herein, or industry-standard test methods if no test method is expressly disclosed.

The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document cited herein, the meaning or definition assigned to that term in this document shall govern.

In this document, including the examples and claims, a statement that something is "based on" something else should be understood to mean that it is determined at least in part by the thing that it is indicated as being based on. To indicate that something must be completely determined based on something else, it is described as being "based EXCLUSIVELY on" whatever it must be completely determined by.

## Claims

1. An automated system for evaluating Multisystem Inflammatory Syndrome in Children (MIS-C) associated with SARS-CoV-2, the system comprising:
a light source (312) configured to irradiate cells of a body fluid sample;
one or more light scatter detector units (350A) configured to measure light scattered from the light source scattered by the cells in the body fluid sample;
a data processing module (304) comprising a processor and a non-transitory computer readable medium, wherein the non-transitory computer readable medium stores instructions that, when executed by the processor, configure the processor to perform a method comprising:
analyzing a monocyte or granulocyte cell population parameter determined for the body fluid sample based on data from the one or more light scatter detector units; and
evaluating MIS-C associated with SARS-CoV-2 based at least in part on the monocyte or granulocyte population parameter.

2. The system of claim 1, wherein analyzing the monocyte or granulocyte cell population parameter comprises:
a) analyzing standard deviation of neutrophil volume;
b) analyzing standard deviation of neutrophil volume and mean of the median angle light scatter of early granulated cells (EGC_LMALS_MEAN); or
c) comparing one or more cell population parameters comprising the monocyte or granulocyte cell population parameter to a threshold.

3. The system of claim 1 or 2, wherein the system comprises an electrode assembly (334, 336) configured to measure direct current (DC) impedance of cells of the body fluid sample.

4. A method for evaluating MIS-C associated with SARS-CoV-2, the method comprising:
flowing a body fluid sample through a flowcell;
irradiating a plurality of cells in the body fluid sample in the flowcell;
measuring light scatter from individual cells of the plurality of cells;
analyzing a monocyte or granulocyte cell population parameter determined for the body fluid sample based on the light scatter; and
evaluating MIS-C associated with SARS-CoV-2 based at least in part on the monocyte or granulocyte population parameter.

5. The method of claim 4, wherein the body fluid sample is whole blood.

6. The system of any one of claims 1-3 or the method of claim 4 or claim 5, wherein analyzing the monocyte or granulocyte cell population parameter comprises analyzing a monocyte cell population parameter, optionally wherein the monocyte cell population parameter comprises monocyte distribution width (MDW).

7. The system of any one of claims 1-3 or 6 or the method of any one of claims 4-6, wherein analyzing the monocyte or granulocyte cell population parameter comprises analyzing a granulocyte cell population parameter, optionally wherein the granulocyte cell population parameter comprises early granulated cells count (EGC COUNT) or granulocyte count (IG COUNT).

8. The method of any one of claims 4-7, wherein:
a) analyzing the monocyte or granulocyte cell population parameter comprises comparing one or more cell population parameters comprising the monocyte or granulocyte cell population parameter to a threshold range;
b) the method does not subject the sample to dye prior to analysis;
c) the MIS-C associated with SARS-CoV-2 is suspected based on at least one cell population parameter being beyond a threshold range, optionally wherein the at least one cell population parameter being beyond its threshold range comprises MDW;
d) evaluating MIS-C associated with SARS-CoV-2 based at least in part on the monocyte or granulocyte population parameter comprises determining that a patient from whom the body fluid sample was obtained has or is at risk of developing MIS-C when the monocyte or granulocyte population parameter is above a threshold, optionally wherein the monocyte or granulocyte population parameter comprises MDW, and wherein the threshold for MDW is selected from a group consisting of at least 23 and at least 24; and/or
e) evaluating MIS-C associated with SARS-CoV-2 based at least in part on the monocyte or granulocyte population parameter comprises determining that a patient from whom the body fluid sample was obtained is eligible for discharge or de-escalation when the monocyte or granulocyte population parameter is below a threshold, optionally wherein the monocyte or granulocyte population parameter comprises MDW, and wherein the threshold for MDW is selected from a group consisting of up to 20, up to 21, up to 22, up to 23 and up to 24.

9. The method of any one of claims 8c), 8d) or 8e), wherein the threshold is age adjusted.

10. The method of any one of claims 4-9 wherein:
a) the body fluid sample is acquired from a hospitalized or non-hospitalized patient;
b) MIS-C associated with SARS-Cov-2 is evaluated automatically with a CBC with differential test order; and/or
c) the body fluid sample is acquired from a pediatric patient.

11. The system of any one of claims 1-3, 6 or 7 or the method of any of one of claims 4-10, wherein evaluating MIS-C associated with SARS-CoV-2 based at least in part on the monocyte or granulocyte population parameter comprises:
a) evaluating the monocyte or granulocyte population parameter in combination with an additional parameter recommended for assessing MIS-C, optionally wherein the additional parameter recommended for assessing MIS-C is selected from a group consisting of: complete blood count (CBC), C-reactive protein (CRP), erythrocyte sedimentation rate (ESR), fibrinogen, procalcitonin, d-dimer, ferritin, lactic acid dehydrogenase (LDH), interleukin 6 (IL-6), or albumin, and a combination thereof; or
b) evaluating a combination of parameters comprising at least two parameters selected from the group consisting of: a monocyte cell population parameter, a granulocyte cell population parameter, and a red blood cell population parameter, optionally wherein:
the monocyte cell population parameter is a parameter selected from a group consisting of: monocyte distribution width (MDW), standard deviation of monocyte volume (Mo_DC_SD), mean of monocyte volume (Mo_DC_Mean), standard deviation of axial light loss of monocytes (Mo_All_SD), reactive monocytes (RE-MONO);
the granulocyte cell population parameter is a parameter selected from a group consisting of: mean volume of neutrophils (Ne_DC_Mean), mean lower median angle light scatter of early granulated cells (Egc_Lmals_Mean), mean median angle light scatter of early granulated cells (Egc_Mals_Mean), mean lower median angle light scatter of neutrophils (Ne_Lmals_Mean), mean median angle light scatter of neutrophils (Ne_Mals_Mean), standard deviation in the axial light loss of neutrophils (Ne_All_SD), standard deviation in the neutrophil volume (Ne_DC_SD), mean axial light loss of neutrophils (Ne_All_Mean), standard deviation in the low angle light scatter of neutrophils (Ne_Lals_SD), mean axial light loss of early granulated cells (Egc_All_Mean), the standard deviation in the lower median angle light scatter of neutrophils (Ne_Lmals_SD), mean opacity of early granulated cells (Egc_Op_Mean), standard deviation of upper median angle light scatter of early granulated cells (Egc_Umals_SD), neutrophil granularity (NEUT-GI), neutrophil activation (NEUT-RI), and granulocyte count (IG COUNT);
the red blood cell population parameter is a parameter selected from a group consisting of: red blood cell count (Rbc), hemoglobin (Hgb), hematocrit (Hct), standard deviation in the opacity of non-nucleated red blood cells (NNrbc_Op_SD), mean opacity of mature red blood cells (Nretic_Op_Mean), mean AL2 (a derivative of axial light loss (ALL) which has been scaled and calibrated to compensate for manufacturing variation in optical components) of non-nucleated red blood cells (NrbcAL2Mean), hypochromic red blood cells (HYPO-HE), hyperchromic red blood cells (HYPER-HE), reticulocyte haemoglobin equivalent (RET-HE), nucleated red blood cells (NRBC), microcytic red blood cells (MICROR), macrocytic red blood cells (MACROR), and fragmented red blood cells (FRC).

12. The system of claim 11b) or the method of claim 11b), wherein the combination of parameters comprises a lymphocyte cell population parameter, optionally wherein the lymphocyte cell population parameter is selected from a group consisting of: standard deviation in the lymphocyte volume (Ly_DC_SD), standard deviation in the axial light loss of lymphocytes (Ly_All_SD), standard deviation in the lower median angle light scatter of lymphocytes (Ly_Lmals_SD), standard deviation in the median angle light scatter of lymphocytes (Ly_Mals_SD), standard deviation in the low angle light scatter of lymphocytes (Ly_Lals_SD), standard deviation in the lymphocyte opacity (Ly_Op_SD), reactive lymphocytes (RE-LYMP), and antibody-synthesizing lymphocytes (AS-LYMP).

13. The method of any one of claims 4-12, wherein the method detects MIS-C or detects the severity of MIS-C in a patient, the method comprising:
providing the sample from the patient;
determining the value of the cell population parameter in the patient, wherein the cell population parameter comprises a monocyte or granulocyte population parameter; and
determining whether said patient has an increased risk of suffering MIS-C based on the value of the cell population parameter, optionally wherein the patient is known to have been exposed to SARS-CoV-2 or suspected to have been exposed to SARS-CoV-2.

14. The method of claim 13, wherein when the cell population parameter comprises MDW and the method further comprises comparing the cell population parameter to a threshold value or a threshold range, optionally wherein determining whether said patient has an increased risk of suffering MIS-C based on the value of the cell population parameter comprises:
a. determining that the patient has or is at risk of developing MIS-C when the cell population parameter is greater than the threshold value; or
b. determining the patient is eligible for de-escalation and/or discharge when the cell population parameter is less than the threshold value.

15. The method of claim 13 or claim 14, wherein determining whether said patient has the increased risk of suffering MIS-C based on the value of the cell population parameter comprises evaluating a combination of parameters comprising at least two parameters selected from the group consisting of: a monocyte cell population parameter, a granulocyte cell population parameter, and a red blood cell population parameter.

## Patentansprüche

1. Automatisiertes System zur Bewertung des multisystemischen Entzündungssyndroms bei Kindern (MIS-C) im Zusammenhang mit SARS-CoV-2, wobei das System umfasst:
eine Lichtquelle (312), die dazu konfiguriert ist, Zellen einer Körperflüssigkeitsprobe zu bestrahlen;
eine oder mehrere Streulichtdetektoreinheiten (350A), die dazu konfiguriert sind, Streulicht zu messen, das von der Lichtquelle stammt und von den Zellen in der Körperflüssigkeitsprobe gestreut wird;
ein Datenverarbeitungsmodul (304), umfassend einen Prozessor und ein nichttransitorisches computerlesbares Speichermedium, wobei das nichttransitorische computerlesbare Speichermedium Anweisungen speichert, die, wenn sie von dem Prozessor ausgeführt werden, den Prozessor dazu konfigurieren, ein Verfahren auszuführen, das Folgendes umfasst:
Analysieren eines Monozyten- oder Granulozyten-Zellpopulationsparameters, der für die Körperflüssigkeitsprobe, basierend auf Daten von der einen oder den mehreren Streulichtdetektoreinheiten, bestimmt wurde; und
Bewertung von MIS-C im Zusammenhang mit SARS-CoV-2, zumindest teilweise basierend auf dem Monozyten- oder Granulozytenpopulationsparameter.

2. System nach Anspruch 1, wobei das Analysieren des Monozyten- oder Granulozyten-Zellpopulationsparameters Folgendes umfasst:
a) Analysieren der Standardabweichung des Neutrophilenvolumens;
b) Analysieren der Standardabweichung des Neutrophilenvolumens und des mittleren Werts des Streulichts im mittleren Winkel früher Granulozyten-Zellen (EGC_LMALS_MEAN); oder
c) Vergleichen eines oder mehrerer Zellpopulationsparameter, die den Monozyten- oder Granulozyten-Zellpopulationsparameter umfassen, mit einer Schwelle.

3. System nach Anspruch 1 oder 2, wobei das System eine Elektrodenanordnung (334, 336) umfasst, die dazu konfiguriert ist, die Gleichstrom- (DC) -Impedanz von Zellen der Körperflüssigkeitsprobe zu messen.

4. Verfahren zur Bewertung von MIS-C im Zusammenhang mit SARS-CoV-2, wobei das Verfahren umfasst:
Durchleiten einer Körperflüssigkeitsprobe durch eine Durchflusszelle;
Bestrahlen einer Vielzahl von Zellen in der Körperflüssigkeitsprobe in der Durchflusszelle;
Messen von Streulicht von einzelnen Zellen der Vielzahl von Zellen;
Analysieren eines Monozyten- oder Granulozyten-Zellpopulationsparameters, der für die Körperflüssigkeitsprobe, basierend auf dem Streulicht, bestimmt wurde; und
Bewertung von MIS-C im Zusammenhang mit SARS-CoV-2, zumindest teilweise basierend auf dem Monozyten- oder Granulozytenpopulationsparameter.

5. Verfahren nach Anspruch 4, wobei die Körperflüssigkeitsprobe Vollblut ist.

6. System nach einem der Ansprüche 1-3 oder Verfahren nach Anspruch 4 oder Anspruch 5, wobei das Analysieren des Monozyten- oder Granulozyten-Zellpopulationsparameters das Analysieren eines Monozyten-Zellpopulationsparameters umfasst, wobei der Monozyten-Zellpopulationsparameter optional die Monozytenverteilungsbreite (MDW) umfasst.

7. System nach einem der Ansprüche 1-3 oder 6 oder Verfahren nach einem der Ansprüche 4-6, wobei das Analysieren des Monozyten- oder Granulozyten-Zellpopulationsparameters das Analysieren eines Granulozyten-Zellpopulationsparameters umfasst, wobei der Granulozyten-Zellpopulationsparameter optional eine Zählung früher Granulozyten-Zellen (EGC COUNT) oder eine Granulozytenzahl (IG COUNT) umfasst.

8. Verfahren nach einem der Ansprüche 4-7, wobei:
a) das Analysieren des Monozyten- oder Granulozyten-Zellpopulationsparameters das Vergleichen eines oder mehrerer Zellpopulationsparameter, die den Monozyten- oder Granulozyten-Zellpopulationsparameter umfassen, mit einem Schwellenbereich umfasst;
b) das Verfahren die Probe vor der Analyse keiner Färbung unterzieht;
c) das MIS-C im Zusammenhang mit SARS-CoV-2 vermutet wird, basierend darauf dass mindestens ein Zellpopulationsparameter außerhalb eines Schwellenbereichs liegt, wobei der mindestens eine Zellpopulationsparameter, der außerhalb seines Schwellenbereichs liegt, optional MDW umfasst;
d) die Bewertung von MIS-C im Zusammenhang mit SARS-CoV-2, zumindest teilweise basierend auf dem Monozyten- oder Granulozytenpopulationsparameter, das Feststellen umfasst, dass ein Patient, von dem die Körperflüssigkeitsprobe gewonnen wurde, MIS-C aufweist oder Gefahr läuft, MIS-C zu entwickeln, wenn der Monozyten- oder Granulozytenpopulationsparameter über einer Schwelle liegt, wobei der Monozyten- oder Granulozytenpopulationsparameter optional MDW umfasst, und wobei die Schwelle für MDW aus einer Gruppe ausgewählt ist, die aus mindestens 23 und mindestens 24 besteht; und/oder
e) die Bewertung von MIS-C im Zusammenhang mit SARS-CoV-2, zumindest teilweise basierend auf dem Monozyten- oder Granulozytenpopulationsparameter, das Feststellen umfasst, dass ein Patient, von dem die Körperflüssigkeitsprobe gewonnen wurde, als für eine Entlassung oder Deeskalation in Frage kommend ist, wenn der Monozyten- oder Granulozytenpopulationsparameter unter einer Schwelle liegt, wobei der Monozyten- oder Granulozytenpopulationsparameter optional MDW umfasst, und wobei die Schwelle für MDW aus einer Gruppe ausgewählt ist, die aus bis zu 20, bis zu 21, bis zu 22, bis zu 23 und bis zu 24 besteht.

9. Verfahren nach einem der Ansprüche 8c), 8d) oder 8e), wobei die Schwelle altersangepasst ist.

10. Verfahren nach einem der Ansprüche 4-9, wobei:
a) die Körperflüssigkeitsprobe von einem hospitalisierten oder nicht hospitalisierten Patienten gewonnen wird;
b) MIS-C im Zusammenhang mit SARS-CoV-2 automatisch mit einem CBC mit Differenzialblutbild bewertet wird; und/oder
c) die Körperflüssigkeitsprobe von einem pädiatrischen Patienten gewonnen wird.

11. System nach einem der Ansprüche 1-3, 6 oder 7 oder Verfahren nach einem der Ansprüche 4-10, wobei die Bewertung von MIS-C im Zusammenhang mit SARS-CoV-2, zumindest teilweise basierend auf dem Monozyten- oder Granulozytenpopulationsparameter, Folgendes umfasst:
a) Bewerten des Monozyten- oder Granulozytenpopulationsparameters in Kombination mit einem zusätzlichen Parameter, der zur Bewertung von MIS-C empfohlen wird, wobei der zusätzliche Parameter, der zur Bewertung von MIS-C empfohlen wird, optional aus einer Gruppe ausgewählt ist, die aus Folgendem besteht:
komplettes Blutbild (CBC), C-reaktives Protein (CRP), Erythrozytensedimentationsrate (ESR), Fibrinogen, Procalcitonin, D-Dimer, Ferritin, Laktatdehydrogenase (LDH), Interleukin 6 (IL-6) oder Albumin, und eine Kombination davon; oder
b) Bewerten einer Kombination von Parametern, die mindestens zwei Parameter umfasst, die aus der Gruppe ausgewählt sind, die aus Folgendem besteht: ein Monozyten-Zellpopulationsparameter, ein Granulozyten-Zellpopulationsparameter und ein Erythrozyten-Zellpopulationsparameter, optional wobei:
der Monozyten-Zellpopulationsparameter ein Parameter ist, der aus einer Gruppe ausgewählt ist, die aus Folgendem besteht:
Monozytenverteilungsbreite (MDW), Standardabweichung des Monozytenvolumens (Mo_DC_SD), mittleres Monozytenvolumen (Mo_DC_Mean), Standardabweichung des axialen Lichtverlusts von Monozyten (Mo_All_SD), reaktive Monozyten (RE-MONO);
der Granulozyten-Zellpopulationsparameter ein Parameter ist, der aus einer Gruppe ausgewählt ist, die aus Folgendem besteht: mittleres Volumen der Neutrophilen (Ne_DC_Mean), mittleres Streulicht im unteren mittleren Winkel früher Granulozyten-Zellen (Egc_Lmals_Mean), mittleres Streulicht im mittleren Winkel früher Granulozyten-Zellen (Egc_Mals_Mean), mittleres Streulicht im unteren mittleren Winkel der Neutrophilen (Ne_Lmals_Mean), mittleres Streulicht im mittleren Winkel der Neutrophilen (Ne_Mals_Mean), Standardabweichung im axialen Lichtverlust der Neutrophilen (Ne_All_SD), Standardabweichung im Neutrophilenvolumen (Ne_DC_SD), mittlerer axialer Lichtverlust der Neutrophilen (Ne_All_Mean), Standardabweichung im Streulicht im kleinen Winkel der Neutrophilen (Ne_Lals_SD), mittlerer axialer Lichtverlust früher Granulozyten-Zellen (Egc_All_Mean), die Standardabweichung im Streulicht im unteren mittleren Winkel der Neutrophilen (Ne_Lmals_SD), mittlere Opazität früher Granulozyten-Zellen (Egc_Op_Mean), Standardabweichung des Streulichts im oberen mittleren Winkel früher Granulozyten-Zellen (Egc_Umals_SD), neutrophile Granularität (NEUT-GI), neutrophile Aktivierung (NEUT-RI) und Granulozytenzahl (IG COUNT);
der Erythrozyten-Zellpopulationsparameter ein Parameter ist, der aus einer Gruppe ausgewählt ist, die aus Folgendem besteht: Erythrozytenzahl (Rbc), Hämoglobin (Hgb), Hämatokrit (Hct), Standardabweichung in der Opazität nicht kernhaltiger Erythrozyten (NNrbc_Op_SD), mittlere Opazität reifer Erythrozyten (Nretic_Op_Mean), mittleres AL2 (ein Derivat des axialen Lichtverlusts (ALL), das skaliert und kalibriert wurde, um Fertigungsvariationen in optischen Komponenten zu kompensieren) nicht kernhaltiger Erythrozyten (NrbcAL2Mean), hypochrome Erythrozyten (HYPO-HE), hyperchrome Erythrozyten (HYPER-HE), Retikulozytenhämoglobinäquivalent (RET-HE), kernhaltige Erythrozyten (NRBC), mikrozytäre Erythrozyten (MICROR), makrozytäre Erythrozyten (MACROR) und fragmentierte Erythrozyten (FRC).

12. System nach Anspruch 11b) oder Verfahren nach Anspruch 11b), wobei die Parameterkombination einen Lymphozyten-Zellpopulationsparameter umfasst, wobei der Lymphozyten-Zellpopulationsparameter optional aus einer Gruppe ausgewählt ist, die aus Folgendem besteht: Standardabweichung im Lymphozytenvolumen (Ly_DC_SD), Standardabweichung im axialen Lichtverlust von Lymphozyten (Ly_All_SD), Standardabweichung im Streulicht im unteren mittleren Winkel von Lymphozyten (Ly_Lmals_SD), Standardabweichung im Streulicht im mittleren Winkel von Lymphozyten (Ly_Mals_SD), Standardabweichung im Streulicht im kleinen Winkel von Lymphozyten (Ly_Lals_SD), Standardabweichung in der Lymphozytenopazität (Ly_Op_SD), reaktive Lymphozyten (RE-LYMP) und Antikörpersynthetisierende Lymphozyten (AS-LYMP).

13. Verfahren nach einem der Ansprüche 4-12, wobei das Verfahren MIS-C detektiert oder den Schweregrad von MIS-C bei einem Patienten detektiert, das Verfahren umfassend:
Bereitstellen der Probe von dem Patienten;
Bestimmen des Werts des Zellpopulationsparameters bei dem Patienten, wobei der Zellpopulationsparameter einen Monozyten- oder Granulozytenpopulationsparameter umfasst; und
Bestimmen, ob der genannte Patient ein erhöhtes Risiko, an MIS-C zu erkranken, basierend auf dem Wert des Zellpopulationsparameters, aufweist, wobei bei dem Patienten optional bekannt ist, dass er SARS-CoV-2 ausgesetzt gewesen ist, oder vermutet wird, dass er SARS-CoV-2 ausgesetzt gewesen ist.

14. Verfahren nach Anspruch 13, wobei, wenn der Zellpopulationsparameter MDW umfasst und das Verfahren weiter das Vergleichen des Zellpopulationsparameters mit einem Schwellenwert oder einem Schwellenbereich umfasst, wobei das Bestimmen, ob der genannte Patient ein erhöhtes Risiko, an MIS-C zu erkranken, basierend auf dem Wert des Zellpopulationsparameters aufweist, optional Folgendes umfasst:
a. Feststellen, dass der Patient MIS-C aufweist oder Gefahr läuft, MIS-C zu entwickeln, wenn der Zellpopulationsparameter größer als der Schwellenwert ist; oder
b. Feststellen, dass der Patient als für eine Deeskalation und/oder Entlassung in Frage kommend ist, wenn der Zellpopulationsparameter kleiner als der Schwellenwert ist.

15. Verfahren nach Anspruch 13 oder Anspruch 14, wobei das Bestimmen, ob der genannte Patient das erhöhte Risiko, an MIS-C zu erkranken, basierend auf dem Wert des Zellpopulationsparameters aufweist, das Bewerten einer Kombination von Parametern umfasst, die mindestens zwei Parameter umfasst, die aus der Gruppe ausgewählt sind, die aus Folgendem besteht: ein Monozyten-Zellpopulationsparameter, ein Granulozyten-Zellpopulationsparameter und ein Erythrozyten-Zellpopulationsparameter.

## Revendications

1. Système automatisé d'évaluation du syndrome inflammatoire multisystémique chez l'enfant (MIS-C) associé au SARS-CoV-2, le système comprenant :
une source lumineuse (312) configurée pour irradier des cellules d'un échantillon de fluide corporel ;
une ou plusieurs unités de détection de diffusion de la lumière (350A) configurées pour mesurer la lumière diffusée provenant de la source lumineuse et diffusée par les cellules dans l'échantillon de fluide corporel ;
un module de traitement de données (304) comprenant un processeur et un support informatique non transitoire lisible par ordinateur, dans lequel le support informatique non transitoire lisible par ordinateur stocke des instructions qui, lorsqu'elles sont mises en œuvre par le processeur, configurent le processeur pour exécuter un procédé comprenant :
l'analyse d'un paramètre de population cellulaire de monocytes ou de granulocytes déterminé pour l'échantillon de fluide corporel sur la base de données provenant des une ou plusieurs unités de détection de diffusion de la lumière ; et
l'évaluation du MIS-C associé au SARS-CoV-2 sur la base, au moins en partie, du paramètre de population de monocytes ou de granulocytes.

2. Système selon la revendication 1, dans lequel l'analyse du paramètre de population cellulaire de monocytes ou de granulocytes comprend :
a) l'analyse de l'écart-type du volume des neutrophiles ;
b) l'analyse de l'écart-type du volume des neutrophiles et de la moyenne de la diffusion de la lumière à angle médian de cellules granuleuses précoces (EGC_LMALS_MEAN) ; ou
c) la comparaison d'un ou plusieurs paramètres de population cellulaire comprenant le paramètre de population cellulaire de monocytes ou de granulocytes à un seuil.

3. Système selon la revendication 1 ou revendication 2, dans lequel le système comprend un ensemble d'électrodes (334, 336) configuré pour mesurer l'impédance en courant continu (CC) de cellules de l'échantillon de fluide corporel.

4. Procédé d'évaluation du MIS-C associé au SARS-CoV-2, le procédé comprenant :
la circulation d'un échantillon de fluide corporel à travers une cellule d'écoulement ;
l'irradiation d'une pluralité de cellules dans l'échantillon de fluide corporel dans la cellule d'écoulement ;
la mesure de la diffusion de la lumière à partir de cellules individuelles de la pluralité de cellules ;
l'analyse d'un paramètre de population cellulaire de monocytes ou de granulocytes déterminé pour l'échantillon de fluide corporel sur la base de la diffusion de la lumière ; et
l'évaluation du MIS-C associé au SARS-CoV-2 sur la base, au moins en partie, du paramètre de population de monocytes ou de granulocytes.

5. Procédé selon la revendication 4, dans lequel l'échantillon de fluide corporel est du sang total.

6. Système selon l'une quelconque des revendications 1 à 3 ou procédé selon la revendication 4 ou revendication 5, dans lequel l'analyse du paramètre de population cellulaire de monocytes ou de granulocytes comprend l'analyse d'un paramètre de population cellulaire de monocytes, facultativement dans lequel le paramètre de population cellulaire de monocytes comprend la largeur de distribution des monocytes (MDW).

7. Système selon l'une quelconque des revendications 1 à 3 ou 6 ou procédé selon l'une quelconque des revendications 4 à 6, dans lequel l'analyse du paramètre de population cellulaire de monocytes ou de granulocytes comprend l'analyse d'un paramètre de population cellulaire de granulocytes, facultativement dans lequel le paramètre de population cellulaire de granulocytes comprend une numération de cellules granuleuses précoces (EGC COUNT) ou une numération de granulocytes (IG COUNT).

8. Procédé selon l'une quelconque des revendications 4 à 7, dans lequel :
a) l'analyse du paramètre de population cellulaire de monocytes ou de granulocytes comprend la comparaison d'un ou plusieurs paramètres de population cellulaire comprenant le paramètre de population cellulaire de monocytes ou de granulocytes à une plage de seuils ;
b) le procédé ne soumet pas l'échantillon à un colorant avant analyse ;
c) le MIS-C associé au SARS-CoV-2 est suspecté sur la base d'au moins un paramètre de population cellulaire se situant au-delà d'une plage de seuils, facultativement dans lequel l'au moins un paramètre de population cellulaire se situant au-delà de sa plage de seuils comprend la MDW ;
d) l'évaluation du MIS-C associé au SARS-CoV-2 sur la base, au moins en partie, du paramètre de population de monocytes ou de granulocytes comprend la détermination qu'un patient duquel l'échantillon de fluide corporel a été obtenu présente ou est à risque de développer le MIS-C lorsque le paramètre de population de monocytes ou de granulocytes est supérieur à un seuil, facultativement dans lequel le paramètre de population de monocytes ou de granulocytes comprend la MDW, et dans lequel le seuil pour la MDW est sélectionné dans un groupe consistant en au moins 23 et au moins 24 ; et/ou
e) l'évaluation du MIS-C associé au SARS-CoV-2 sur la base, au moins en partie, du paramètre de population de monocytes ou de granulocytes comprend la détermination qu'un patient duquel l'échantillon de fluide corporel a été obtenu remplit les critères de sortie ou de désescalade thérapeutique lorsque le paramètre de population de monocytes ou de granulocytes est inférieur à un seuil, facultativement dans lequel le paramètre de population de monocytes ou de granulocytes comprend la MDW, et dans lequel le seuil pour la MDW est sélectionné dans un groupe consistant en jusqu'à 20, jusqu'à 21, jusqu'à 22, jusqu'à 23 et jusqu'à 24.

9. Procédé selon l'une quelconque des revendications 8c), 8d) ou 8e), dans lequel le seuil est ajusté en fonction de l'âge.

10. Procédé selon l'une quelconque des revendications 4 à 9 dans lequel :
a) l'échantillon de fluide corporel est obtenu à partir d'un patient hospitalisé ou non hospitalisé ;
b) le MIS-C associé au SARS-CoV-2 est évalué automatiquement avec une ordonnance de numération formule sanguine (NFS) avec différentiel ; et/ou
c) l'échantillon de fluide corporel est prélevé chez un patient pédiatrique.

11. Système selon l'une quelconque des revendications 1 à 3, 6 ou 7 ou procédé selon l'une quelconque des revendications 4 à 10, dans lequel l'évaluation du MIS-C associé au SARS-CoV-2 sur la base, au moins en partie, du paramètre de population de monocytes ou de granulocytes comprend :
a) l'évaluation du paramètre de population de monocytes ou de granulocytes en combinaison avec un paramètre supplémentaire recommandé pour l'évaluation du MIS-C, facultativement dans lequel le paramètre supplémentaire recommandé pour l'évaluation du MIS-C est sélectionné dans un groupe consistant en :
numération formule sanguine (NFS), protéine C-réactive (CRP), vitesse de sédimentation érythrocytaire (ESR), fibrinogène, procalcitonine, d-dimère, ferritine, déshydrogénase lactique (LDH), interleukine 6 (IL-6) ou albumine, et une combinaison de ceux-ci ; ou
b) l'évaluation d'une combinaison de paramètres comprenant au moins deux paramètres sélectionnés dans le groupe consistant en : un paramètre de population cellulaire de monocytes, un paramètre de population cellulaire de granulocytes, et un paramètre de population de globules rouges, facultativement dans lequel :
le paramètre de population cellulaire de monocytes est un paramètre sélectionné dans un groupe consistant en : largeur de distribution des monocytes (MDW), écart-type du volume des monocytes (Mo_DC_SD), moyenne du volume des monocytes (Mo_DC_Mean), écart-type de la perte de lumière axiale des monocytes (Mo_All_SD), monocytes réactifs (RE-MONO) ;
le paramètre de population cellulaire de granulocytes est un paramètre sélectionné dans un groupe consistant en :
volume moyen des neutrophiles (Ne_DC_Mean), moyenne de la diffusion de la lumière à angle médian inférieur de cellules granuleuses précoces (Egc_Lmals_Mean), moyenne de la diffusion de la lumière à angle médian de cellules granuleuses précoces (Egc_Mals_Mean), moyenne de la diffusion de la lumière à angle médian inférieur des neutrophiles (Ne_Lmals_Mean), moyenne de la diffusion de la lumière à angle médian des neutrophiles (Ne_Mals_Mean), écart-type de la perte de lumière axiale des neutrophiles (Ne_All_SD), écart-type du volume des neutrophiles (Ne_DC_SD), moyenne de la perte de lumière axiale des neutrophiles (Ne_All_Mean), écart-type de la diffusion de la lumière à faible angle des neutrophiles (Ne_Lals_SD), moyenne de la perte de lumière axiale de cellules granuleuses précoces (Egc_All_Mean), écart-type de la diffusion de la lumière à angle médian inférieur des neutrophiles (Ne_Lmals_SD), opacité moyenne de cellules granuleuses précoces (Egc_Op_Mean), écart-type de la diffusion de la lumière à angle médian supérieur de cellules granuleuses précoces (Egc_Umals_SD), granularité des neutrophiles (NEUT-GI), activation des neutrophiles (NEUT- RI), et numération de granulocytes (IG COUNT) ;
le paramètre de population de globules rouges est un paramètre sélectionné dans un groupe consistant en :
numération de globules rouges (Rbc), hémoglobine (Hgb), hématocrite (Hct), écart-type de l'opacité de globules rouges non nucléés (NNrbc_Op_SD), opacité moyenne de globules rouges matures (Nretic_Op_Mean), moyenne d'AL2 (un dérivé de la perte de lumière axiale (ALL) qui a été mis à l'échelle et étalonné pour compenser une variation de fabrication dans des composants optiques) de globules rouges non nucléés (NrbcAL2Mean), globules rouges hypochromes (HYPO-HE), globules rouges hyperchromes (HYPER-HE), équivalent d'hémoglobine réticulocytaire (RET-HE), globules rouges nucléés (NRBC), globules rouges microcytaires (MICROR), globules rouges macrocytaires (MACROR) et globules rouges fragmentés (FRC).

12. Système selon la revendication 11b) ou procédé selon la revendication 11b), dans lequel la combinaison de paramètres comprend un paramètre de population cellulaire de lymphocytes, facultativement dans lequel le paramètre de population cellulaire de lymphocytes est sélectionné dans un groupe consistant en :
écart-type du volume des lymphocytes (Ly_DC_SD), écart-type de la perte de lumière axiale des lymphocytes (Ly_All_SD), écart-type de la diffusion de la lumière à angle médian inférieur des lymphocytes (Ly_Lmals_SD), écart-type de la diffusion de la lumière à angle médian des lymphocytes (Ly_Mals_SD),
écart-type de la diffusion de la lumière à faible angle des lymphocytes (Ly_Lals_SD), écart-type de l'opacité des lymphocytes (Ly_Op_SD), lymphocytes réactifs (RE-LYMP) et lymphocytes synthétisant des anticorps (AS-LYMP).

13. Procédé selon l'une quelconque des revendications 4 à 12, dans lequel le procédé détecte le MIS-C ou détecte la gravité du MIS-C chez un patient, le procédé comprenant :
la fourniture de l'échantillon provenant du patient ;
la détermination de la valeur du paramètre de population cellulaire chez le patient, dans lequel le paramètre de population cellulaire comprend un paramètre de population de monocytes ou de granulocytes ; et
le fait de déterminer si ledit patient présente un risque accru de souffrir du MIS-C sur la base de la valeur du paramètre de population cellulaire, facultativement dans lequel le patient est connu pour avoir été exposé au SARS-CoV-2 ou suspecté d'avoir été exposé au SARS-CoV-2.

14. Procédé selon la revendication 13, dans lequel, lorsque le paramètre de population cellulaire comprend la MDW et que le procédé comprend en outre la comparaison du paramètre de population cellulaire à une valeur seuil ou à une plage de seuils, facultativement dans lequel le fait de déterminer si ledit patient présente un risque accru de souffrir du MIS-C sur la base de la valeur du paramètre de population cellulaire comprend :
a. la détermination que le patient présente ou est à risque de développer le MIS-C lorsque le paramètre de population cellulaire est supérieur à la valeur seuil ; ou
b. la détermination que le patient remplit les critères de désescalade thérapeutique et/ou de sortie lorsque le paramètre de population cellulaire est inférieur à la valeur seuil.

15. Procédé selon la revendication 13 ou revendication 14, dans lequel le fait de déterminer si ledit patient présente le risque accru de souffrir du MIS-C sur la base de la valeur du paramètre de population cellulaire comprend l'évaluation d'une combinaison de paramètres comprenant au moins deux paramètres sélectionnés dans le groupe consistant en : un paramètre de population cellulaire de monocytes, un paramètre de population cellulaire de granulocytes, et un paramètre de population de globules rouges.
